# EUROPEAN PATENT APPLICATION

(11) **EP 4 685 138 A1**
(43) Date of publication of application: **28.01.2026**
(21) Application number: 24190205.5
(22) Date of filing: 23.07.2024
(51) Int. Cl.: C07D 231/12, C07D 231/16, C07D 233/56, C07D 223/08, C07C 249/12, C07K 1/06, C07K 5/06, C07B 43/06

(54) **PROCESS FOR PREPARING PROTECTED AMINES, PYRAZOLOCARBONYL-PROTECTED AMINES AND PEPTIDES**

(71) Applicant: Universiteit Antwerpen, 2000 Antwerpen (BE)
(72) Inventor: MAES, Bert, 2020 Antwerpen (BE); WEEMAES, Karel, 2020 Antwerpen (BE)
(74) Representative: Arnold & Siedsma

(57) **Abstract**

The present invention relates to the field of synthetic organic chemistry, in particular to a process for preparing protected amines, such as blocked isocyanates and protected amino acids or amino acid esters. More in particular, the present invention relates to a process which is safer, practical and more environmentally friendly compared to those in the state of the art, and that avoids the use of phosgene, directly or indirectly, and free isocyanates. The present invention further relates to protected amines obtainable by the method, in particular to protected amino acids and amino acid esters. The present invention also relates to protected oligopeptides, and a method to prepare them starting from protected amino acids or amino acid esters.

## Description

### FIELD OF THE INVENTION

The present invention relates to the field of synthetic organic chemistry, in particular to a process for preparing protected amines, such as blocked isocyanates and protected amino acids or amino acid esters. More in particular, the present invention relates to a process which is safer, practical and more environmentally friendly compared to those in the state of the art, and that avoids the use of phosgene, directly or indirectly, and free isocyanates. The present invention further relates to protected amines obtainable by the method, in particular to protected amino acids and amino acid esters. The present invention also relates to protected oligopeptides, and a method to prepare them starting from protected amino acids or amino acid esters.

### BACKGROUND TO THE INVENTION

Phosgene is a highly toxic, colorless gas, known to lead to pulmonary oedema when inhaled. Despite its known toxicity, and the risk it poses to workers being exposed to it, phosgene is still the prevalent one carbon (1C) synthon used to prepare compounds containing aminocarbonyl moieties, such as urea, carbamate or isocyanate moieties. Phosgene can be used directly, or indirectly by transforming phosgene first into derivatives such as diphosgene, methyl chloroformate, or carbonyldiimidazole, and subsequently using these derivatives to construct the urea, carbamate or isocyanate moieties. Triphosgene, while being a solid at room temperature, also poses a threat to workers due to its toxicity and relatively high vapor pressure. Moreover, when diphosgene or triphosgene are used, phosgene is released in situ.

A well-known example of compounds being prepared from phosgene are isocyanates, such as toluene diisocyanate (TDI) or methylene diphenyl diisocyanate (MDI). Polyurethanes are prepared at an industrial level by reaction of these isocyanates with polyols. On top of the toxicity of phosgene used to prepare them, isocyanates themselves are highly reactive and toxic. To mitigate this reactivity and toxicity, 'blocked isocyanates' were designed, wherein the isocyanate functionality is blocked by a blocking group. Blocked isocyanates can be handled safely by the end user, which could then activate these blocked isocyanates by a trigger such as heat or an additive, providing the high reactivity of the isocyanate only when required. A drawback of blocked isocyanates in the state of the art is that these are mainly prepared from the reaction of free isocyanates with a blocking agent, thereby still requiring the use of phosgene to prepare the free isocyanates at some point in the manufacturing process.

Another example of compounds that are prepared indirectly from phosgene, are activated amino acid esters. Suppo et al. reported the synthesis of activated amino acid esters by reaction of an amino acid ester with carbonyldiimidazole. Besides the drawback of using a phosgene derivative in its preparation, this method does not tolerate the presence of free carboxylic acid groups. Hence, there is currently an industrial need to provide a novel synthetic methodology for obtaining protected amines, in particular for obtaining blocked isocyanates, protected amino acids, or protected amino acid esters, which does not involve the use of phosgene or its derivatives, and which overcomes one or more drawbacks of the state of the art.

### SUMMARY OF THE INVENTION

In a first aspect, the present invention provides a method for preparing a protected amine, the method comprising the steps of:
a) providing an organic carbonate A represented by formula (I) 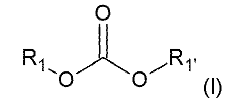 wherein
   R₁ and R_{1'} are each independently selected from any substituted or unsubstituted alkyl, any substituted or unsubstituted alkenyl, any substituted or unsubstituted cycloalkyl, and any substituted or unsubstituted aryl; or
   R₁ and R_{1'}, together with the -O-C-O- fragment to which they are attached, form one or more cyclic structures;
   with the proviso that R₁ and R_{1'} are not -CCl₃;
b) reacting, in a liquid medium, the organic carbonate A provided in step a) with an active hydrogen compound B, thereby obtaining a precursor C; wherein the active hydrogen compound B is selected from substituted or unsubstituted azoles, substituted or unsubstituted secondary amides, substituted or unsubstituted lactams, and substituted or unsubstituted oximes, preferably substituted or unsubstituted azoles, more preferably substituted or unsubstituted pyrazole; and
c) reacting the precursor C obtained in step b) with an amine D having one or more -NH₂ groups, thereby obtaining the protected amine.

In an embodiment, the present invention provides the method as defined herein, wherein the organic carbonate A is selected from diphenyl carbonate, dimethyl carbonate, diethyl carbonate, ethylene carbonate, propylene carbonate and trimethylene carbonate; in particular wherein the organic carbonate A is diphenyl carbonate.

In a further embodiment, the present invention provides the method as defined herein, wherein the active hydrogen compound B is selected from ε-caprolactam, 3,5-dimethylpyrazole, acetoxime, and 2-butanone oxime.

In a further embodiment, the present invention provides the method as defined herein, wherein the active hydrogen compound B is represented by formula (II) 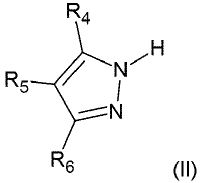 wherein
R₄, and R₆ are each independently selected from hydrogen, any halogen, any substituted or unsubstituted alkyl, any substituted or unsubstituted alkenyl, any substituted or unsubstituted cycloalkyl, and any substituted or unsubstituted aryl; and
R₅ is selected from hydrogen, hydroxy, thio, any substituted or unsubstituted amino, any substituted or unsubstituted alkoxy or aryloxy, any substituted or unsubstituted alkyl, any substituted or unsubstituted alkenyl, any substituted or unsubstituted cycloalkyl, any substituted or unsubstituted aryl, any substituted or unsubstituted ester, any substituted or unsubstituted carbonyl, any substituted or unsubstituted amide, any substituted or unsubstituted carbonate, and any substituted or unsubstituted carbamate.

In a further embodiment, the present invention provides the method as defined herein, wherein the amine D is selected from any monoamine, any diamine, any triamine, and any polyamine; preferably the amine D is an amino acid, an amino acid ester, or an alkanol amine; more preferably the amine D is an alpha-amino acid or an alpha-amino acid ester.

In a further embodiment, the present invention provides the method as defined herein, wherein the amine D is an alpha-amino acid, an alpha-amino acid ester, or an alpha-amino acid residue connected to a solid support, represented by formula (III) 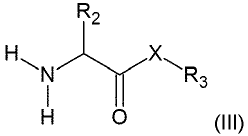 wherein
R₂ is selected from -H, -OH, -SH, SeH, -NR_{b}R_{b}, -C₁₋₁₂alkyl, -C₂₋₁₂alkenyl, -C₃₋₇cycloalkyl, - CHO, -C(O)R_{c}, -COOH, -OC(O)R_{c}, -C(O)OR_{c}, -OC(O)OR_{c}, -NR_{b}C(O)R_{c}, -C(O)NR_{b}R_{b}, - NR_{b}C(O)OR_{c}, and -OC(O)NR_{b}R_{b}-, and Ar₂; wherein said -C₁₋₁₂alkyl, -C₂₋₁₂alkenyl, and -C₃₋₇cycloalkyl optionally comprises one or more heteroatoms selected from N, O, and S and/or is substituted with one or more substituents selected from -H, -OH, -SH, -NR_{d}R_{d'}, -C₁₋₁₂alkyl, -C₂₋₁₂alkenyl, -C₃₋₇cycloalkyl, -CHO, -C(O)Rₑ, -COOH, -OC(O)Rₑ, -C(O)ORₑ, -OC(O)ORₑ, - NR_{d}C(O)Rₑ, -C(O)NR_{d}R_{d'}, -NR_{d}C(O)ORₑ, -OC(O)NR_{d}R_{d'}-, and Ar₃;
each instance of R_{b}, each instance of R_{b'}, each instance of R_{c}, each instance of R_{d}, each instance of R_{d'} and each instance of Rₑ is independently selected from -H, and -C₁₋₁₂alkyl; Ar₂ and Ar₃ are each independently a 5- to 10-membered aromatic cycle optionally comprising one or more heteroatoms selected from O, N and S and/or substituted with one or more substituents independently selected from -halogen, -C₁₋₆alkyl, -C₂₋₆alkenyl, -C₃₋₇cycloalkyl, -OH, -SH, NR_{f}R_{f'}, and -OC₁₋₆alkyl;
each instance of R_{f} and each instance of R_{f'} is independently selected from -H, and -C₁₋₆alkyl;
X is selected from -O-, and -NH-; and
R₃ is selected from -H, and -C₁₋₁₂alkyl, or R₃ is a solid support.

In a further embodiment, the present invention provides the method as defined herein, wherein step b) is performed in the presence of a nucleophilic catalyst.

In a further embodiment, the present invention provides the method as defined herein, wherein in step b) the liquid medium is the organic carbonate A or a solvent.

In a further aspect, the present invention provides a protected amine, in particular a protected amino acid or amino acid ester, represented by formula (IV) 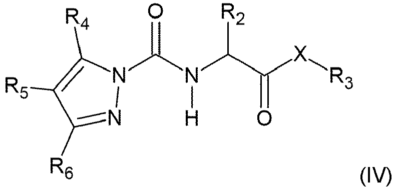 wherein
R₂ is selected from -H, -OH, -SH, SeH, -NR_{b}R_{b}, -C₁₋₁₂alkyl, -C₂₋₁₂alkenyl, -C₃₋₇cycloalkyl, - CHO, -C(O)R_{c}, -COOH, -OC(O)R_{c}, -C(O)OR_{c}, -OC(O)OR_{c}, -NR_{b}C(O)R_{c}, -C(O)NR_{b}R_{b}, - NR_{b}C(O)OR_{c}, and -OC(O)NR_{b}R_{b'}-, and Ar₂; wherein said -C₁₋₁₂alkyl, -C₂₋₁₂alkenyl, and -C₃₋₇cycloalkyl optionally comprises one or more heteroatoms selected from N, O, and S and/or is substituted with one or more substituents selected from -H, -OH, -SH, -NR_{d}R_{d'}, -C₁₋₁₂alkyl, -C₂₋₁₂alkenyl, -C₃₋₇cycloalkyl, -CHO, -C(O)Rₑ, -COOH, -OC(O)Rₑ, -C(O)ORₑ, -OC(O)ORₑ, - NR_{d}C(O)Rₑ, -C(O)NR_{d}R_{d'}, -NR_{d}C(O)ORₑ, -OC(O)NR_{d}R_{d'}-, and Ar₃;
each instance of R_{b}, each instance of R_{b'}, each instance of R_{c}, each instance of R_{d}, each instance of R_{d'} and each instance of Rₑ is independently selected from -H, and -C₁₋₁₂alkyl; Ar₂ and Ar₃ are each independently a 5- to 10-membered aromatic cycle optionally comprising one or more heteroatoms selected from O, N and S and/or substituted with one or more substituents independently selected from -halogen, -C₁₋₆alkyl, -C₂₋₆alkenyl, -C₃₋₇cycloalkyl, -OH, -SH, NR_{f}R_{f'}, and -OC₁₋₆alkyl;
each instance of R_{f} and each instance of R_{f'} is independently selected from -H, and -C₁₋₆alkyl;
X is selected from -O-, and -NH-;
R₃ is selected from -H, and -C₁₋₁₂alkyl, or R₃ is a solid support;
R₄, and R₆ are each independently selected from hydrogen, any halogen, any substituted or unsubstituted alkyl, any substituted or unsubstituted alkenyl, any substituted or unsubstituted cycloalkyl, and any substituted or unsubstituted aryl; and R₅ is selected from hydrogen, hydroxy, thio, any substituted or unsubstituted amino, any substituted or unsubstituted alkoxy or aryloxy, any substituted or unsubstituted alkyl, any substituted or unsubstituted alkenyl, any substituted or unsubstituted cycloalkyl, any substituted or unsubstituted aryl, any substituted or unsubstituted ester, any substituted or unsubstituted carbonyl, any substituted or unsubstituted amide, any substituted or unsubstituted carbonate, and any substituted or unsubstituted carbamate.

In an embodiment, the present invention provides the protected amine as defined herein, wherein the protected amine is a protected alpha-amino acid represented by formula (IVa) 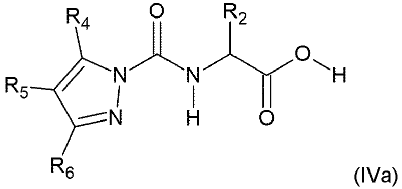 wherein
R₂ is selected from -H, -OH, -SH, SeH, -NR_{b}R_{b'}, -C₁₋₁₂alkyl, -C₂₋₁₂alkenyl, -C₃₋₇cycloalkyl, - CHO, -C(O)R_{c}, -COOH, -OC(O)R_{c}, -C(O)OR_{c}, -OC(O)OR_{c}, -NR_{b}C(O)R_{c}, -C(O)NR_{b}R_{b}, - NR_{b}C(O)OR_{c}, and -OC(O)NR_{b}R_{b'}-, and Ar₂; wherein said -C₁₋₁₂alkyl, -C₂₋₁₂alkenyl, and -C₃₋₇cycloalkyl optionally comprises one or more heteroatoms selected from N, O, and S and/or is substituted with one or more substituents selected from -H, -OH, -SH, -NR_{d}R_{d'}, -C₁₋₁₂alkyl, -C₂₋₁₂alkenyl, -C₃₋₇cycloalkyl, -CHO, -C(O)Rₑ, -COOH, -OC(O)Rₑ, -C(O)ORₑ, -OC(O)ORₑ, - NR_{d}C(O)Rₑ, -C(O)NR_{d}R_{d'}, -NR_{d}C(O)ORₑ, -OC(O)NR_{d}R_{d'}-, and Ar₃;
R₄ and R₆ are each independently selected from -H, -halogen, -C₁₋₁₂alkyl, -C₂₋₁₂alkenyl, -C₃₋₇cycloalkyl, and Ar₄; wherein said -C₁₋₁₂alkyl, -C₂₋₁₂alkenyl, and -C₃₋₇cycloalkyl optionally comprises one or more heteroatoms selected from N, O, and S and/or is substituted with one or more substituents selected from -halogen;
R₅ is selected from -H, -OH, -SH, -NR_{g}R_{g'}, -C₁₋₁₂alkyl, -C₂₋₁₂alkenyl, -C₃₋₇cycloalkyl, -CHO, - C(O)Rₕ, -COOH, -OC(O)Rₕ, -C(O)ORₕ, -OC(O)ORₕ, -NR_{g}C(O)Rₕ, -C(O)NR_{g}R_{g'}, - NR_{g}C(O)ORₕ, and -OC(O)NR_{g}R_{g'}-, and Ar₅; wherein said -C₁₋₁₂alkyl, -C₂₋₁₂alkenyl, and -C₃₋₇cycloalkyl optionally comprises one or more heteroatoms selected from N, O, and S and/or is substituted with one or more substituents selected from -H, -OH, -SH, -NRᵢR_{i'}, -C₁₋₁₂alkyl, -C₂₋₁₂alkenyl, -C₃₋₇cycloalkyl, -CHO, -C(O)Rⱼ, -COOH, -OC(O)Rⱼ, -C(O)ORⱼ, -OC(O)ORⱼ, - NRᵢC(O)Rⱼ, -C(O)NRᵢR_{i'}, -NRᵢC(O)ORⱼ, -OC(O)NRᵢR_{i'}-, and Ar₆;
Ar₂, Ar₃ Ar₄, Ar₅, and Ar₆ are each independently a 5- to 10-membered aromatic cycle optionally comprising one or more heteroatoms selected from O, N and S and/or substituted with one or more substituents independently selected from -halogen, -C₁₋₆alkyl, -C₂₋₆alkenyl, -C₃₋₇cycloalkyl, -OH, -SH, NR_{f}R_{f'}, and -OC₁₋₆alkyl;
each instance of R_{b}, each instance of R_{b'}, each instance of R_{c}, each instance of R_{d}, each instance of R_{d'}, each instance of Rₑ, each instance of R_{g}, each instance of R_{g'}, each instance of Rₕ, each instance of Rᵢ, each instance of R_{i'} and each instance of Rⱼ is independently selected from -H, and -C₁₋₁₂alkyl; and
each instance of R_{f} and each instance of R_{f'} is independently selected from -H, and -C₁₋₆alkyl.

In a further aspect, the present invention provides a method for preparing a protected dipeptide, the method comprising the steps of:
a) providing a protected amine, in particular a protected alpha-amino acid or alpha-amino acid ester, as defined herein; and
b) coupling the protected amine provided in step a) with a further alpha-amino acid compound, thereby obtaining the protected dipeptide.

In a further aspect, the present invention provides a method for preparing a protected oligopeptide or protected polypeptide, the method comprising the steps of preparing the protected dipeptide as defined herein, and comprising the further steps of:
c) deprotecting the C-terminus of the protected dipeptide, obtained in step b);
d) coupling the C-deprotected dipeptide obtained in step c) with a further alpha-amino acid compound, thereby obtaining the protected oligopeptide; and
e) optionally repeating steps c) and d) one or more times to add further amino acid residues to the protected oligopeptide obtained in step d).

In a further aspect, the present invention provides a method for preparing a protected oligopeptide or protected polypeptide, the method comprising the steps of preparing the protected dipeptide as defined herein, and comprising the further steps of:
c) deprotecting the N-terminus of the protected dipeptide, obtained in step b), by contacting the protected dipeptide with a source of fluoride or hydroxide soluble in organic media, and subsequently with a nucleophile;
d) coupling the deprotected dipeptide obtained in step c) with a further alpha-amino acid compound, thereby obtaining the protected oligopeptide; and
e) optionally repeating steps c) and d) one or more times to add further amino acid residues to the protected oligopeptide obtained in step d).

In a further aspect, the present invention provides a method for preparing an oligopeptide or polypeptide, in particular an oligopeptide or polypeptide with an unprotected N-terminus, the method comprising the steps of preparing the protected oligopeptide or protected polypeptide as defined herein, and comprising the further steps of:
f) contacting the protected oligopeptide or protected polypeptide obtained in step d) or step e) with a source of fluoride or hydroxide soluble in organic media; and
g) contacting the compound obtained in step f) with a nucleophile.

In an embodiment, the present invention provides the method for preparing a protected dipeptide defined herein, or for preparing a protected oligopeptide or protected polypeptide as defined herein, wherein coupling the protected amine, the protected dipeptide, or the protected oligopeptide or protected polypeptide with a further alpha-amino acid is performed in the presence of a carboxylic acid activating agent.

In a further aspect, the present invention provides a protected oligopeptide represented by formula (V) 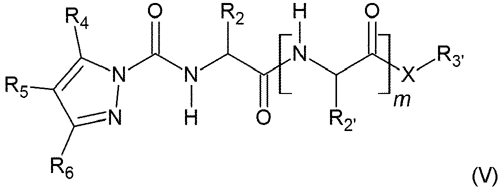 wherein
m is an integer from 1 to 100;
R₂ and each instance of R_{2'} are each independently selected from -H, -OH, -SH, SeH, - NR_{b}R_{b'}, -C₁₋₁₂alkyl, -C₂₋₁₂alkenyl, -C₃₋₇cycloalkyl, -CHO, -C(O)R_{c}, -COOH, -OC(O)R_{c}, - C(O)OR_{c}, -OC(O)OR_{c}, -NR_{b}C(O)R_{c}, -C(O)NR_{b}R_{b'}, -NR_{b}C(O)OR_{c}, and -OC(O)NR_{b}R_{b'}-, and Ar₂; wherein said -C₁₋₁₂alkyl, -C₂₋₁₂alkenyl, and -C₃₋₇cycloalkyl optionally comprises one or more heteroatoms selected from N, O, and S and/or is substituted with one or more substituents selected from -H, -OH, -SH, -NR_{d}R_{d'}, -C₁₋₁₂alkyl, -C₂₋₁₂alkenyl, -C₃₋₇cycloalkyl, - CHO, -C(O)Rₑ, -COOH, -OC(O)Rₑ, -C(O)ORₑ, -OC(O)ORₑ, -NR_{d}C(O)Rₑ, -C(O)NR_{d}R_{d'}, - NR_{d}C(O)ORₑ, -OC(O)NR_{d}R_{d'}-, and Ar₃;
each instance of R_{b}, each instance of R_{b'}, each instance of R_{c}, each instance of R_{d}, each instance of R_{d'} and each instance of Rₑ is independently selected from -H, and -C₁₋₁₂alkyl; Ar₂ and Ar₃ are each independently a 5- to 10-membered aromatic cycle optionally comprising one or more heteroatoms selected from O, N and S and/or substituted with one or more substituents independently selected from -halogen, -C₁₋₆alkyl, -C₂₋₆alkenyl, -C₃₋₇cycloalkyl, -OH, -SH, NR_{f}R_{f'}, and -OC₁₋₆alkyl;
each instance of R_{f} and each instance of R_{f'} is independently selected from -H, and -C₁₋₆alkyl;
X is selected from -O-, and -NH-;
R_{3'} is selected from -H, and -C₁₋₁₂alkyl, or R_{3'} is a solid support;
R₄, and R₆ are each independently selected from hydrogen, any halogen, any substituted or unsubstituted alkyl, any substituted or unsubstituted alkenyl, any substituted or unsubstituted cycloalkyl, and any substituted or unsubstituted aryl; and
R₅ is selected from hydrogen, hydroxy, thio, any substituted or unsubstituted amino, any substituted or unsubstituted alkoxy or aryloxy, any substituted or unsubstituted alkyl, any substituted or unsubstituted alkenyl, any substituted or unsubstituted cycloalkyl, any substituted or unsubstituted aryl, any substituted or unsubstituted ester, any substituted or unsubstituted carbonyl, any substituted or unsubstituted amide, any substituted or unsubstituted carbonate, and any substituted or unsubstituted carbamate.

### DETAILED DESCRIPTION OF THE INVENTION

The present invention will now be further described. In the following passages, different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

When describing the compounds/lipids of the present invention, the terms used are to be construed in accordance with the following definitions, unless a context dictates otherwise:
The term "alkyl" by itself or as part of another substituent refers to a fully saturated hydrocarbon of Formula CₓH₂ₓ₊₁ wherein x is a number greater than or equal to 1. Generally, alkyl groups of this invention comprise from 1 to 20 carbon atoms. Alkyl groups may be linear or branched and may be substituted as indicated herein. When a subscript is used herein following a carbon atom, the subscript refers to the number of carbon atoms that the named group may contain. Thus, for example, C₁₋₄alkyl means an alkyl of one to four carbon atoms. Examples of alkyl groups are methyl, ethyl, n-propyl, i-propyl, butyl, and its isomers (e.g. n-butyl, i-butyl and t-butyl); pentyl and its isomers, hexyl and its isomers, heptyl and its isomers, octyl and its isomers, nonyl and its isomers; decyl and its isomers, undecyl and its isomers, dodecyl and its isomers, tridecyl and its isomers, tetradecyl and its isomers, pentadecyl and its isomers, hexadecyl and its isomers, heptadecyl and its isomers, octadecyl and its isomers, nonadecyl and its isomers, eicosanyl and its isomers. The term "optionally substituted alkyl" refers to an alkyl group optionally substituted with one or more substituents (for example 1 to 4 substituents, for example 1, 2, 3, or 4 substituents) at any available point of attachment.

Whenever the term "substituted" is used in the present invention, it is meant to indicate that one or more hydrogens on the atom indicated in the expression using "substituted" is replaced with a selection from the indicated group, provided that the indicated atom's normal valency is not exceeded, and that the substitution results in a chemically stable compound, i.e. a compound that is sufficiently robust to survive isolation to a useful degree of purity from a reaction mixture, and formulation into a therapeutic agent.

Where groups may be optionally substituted, such groups may be substituted once or more, and preferably once, twice or thrice. Non-limiting examples of such substituents are selected from halogen (-halo), hydroxy (-OH), oxo (=O), nitro (-NO₂), amino (-NR'R"), cyano (-CN), alkyl, cycloalkyl, alkenyl, alkynyl, alkoxy or aryloxy (-OR‴), aryl, heteroaryl, carbonyl (-C(O)R^{iv}), carboxyl (-COOH), ester or alkoxycarbonyl (-C(O)OR^{v}), ester or alkylcarbonyloxy (-OC(O)R^{vi}), amido or aminocarbonyl (-NR'C(O)), amido or carbonylamino (-C(O)NR'), heterocyclyl, carbonyl, acyl, arylcarbonyl, thio (-SH), alkylthio (-SR^{vi}), and the like.

The term "alkenyl" or "alkene", as used herein, unless otherwise indicated, means straight-chain, cyclic, or branched-chain hydrocarbon radicals containing at least one carbon-carbon double bond. Examples of alkenyl radicals include ethenyl, E- and Z-propenyl, isopropenyl, E- and Z-butenyl, E- and Z-isobutenyl, E- and Z-pentenyl, E- and Z-hexenyl, E,E-, E,Z-, Z,E-, Z,Z-hexadienyl, be it in the terminal or internal positions, and the like. Generally alkenyl or alkene moieties of the present invention comprise from 2 to 20 C atoms. An optionally substituted alkenyl refers to an alkenyl having optionally one or more substituents (for example 1, 2, 3 or 4), selected from those defined above for substituted alkyl. Unless stated otherwise, when a reference to "alkenyl" or "alkene", it refers to all possible isomers of each of the carbon-carbon double bonds present.

The term "alkynyl", as used herein, unless otherwise indicated, means straight-chain or branched-chain hydrocarbon radicals containing at least one carbon-carbon triple bond. Examples of alkynyl radicals include ethynyl, propynyl, butynyl, pentynyl, hexynyl, hexadiynyl, be it in the terminal or internal positions, and the like. An optionally substituted alkynyl refers to an alkynyl having optionally one or more substituents (for example 1, 2, 3 or 4), selected from those defined above for substituted alkyl.

In the context of the present invention, the alkyl, alkenyl and alkynyl moieties as defined herein may also further comprise one or more heteroatoms, such as selected from N, S or O, in that for example a carbon atom in an alkyl, alkene or alkyne chain is replaced by a heteroatom. When two or more C atoms are replaced by heteroatoms, the heteroatoms may be adjacent or separated, as long as it results in a chemically stable compound, i.e. a compound that is sufficiently robust to survive isolation to a useful degree of purity from a reaction mixture, and formulation into a therapeutic agent. An example of a stable combination of two adjacent heteroatoms is a disulfide (-S-S-) group. Where a carbon atom in an alkyl, alkenyl or alkynyl chain is replaced by an N atom, the N atom may be N or NH depending on the number of bonds connected to said C atom.

The term "cycloalkyl" by itself or as part of another substituent is a cyclic alkyl group, that is to say, a monovalent, saturated, or unsaturated hydrocarbyl group having 1, 2, or 3 cyclic structure. Cycloalkyl includes all saturated or partially saturated (containing 1 or 2 double bonds) hydrocarbon groups containing 1 to 3 rings, including monocyclic, bicyclic, or polycyclic alkyl groups. Cycloalkyl groups may comprise 3 or more carbon atoms in the ring and generally, according to this invention comprise from 3 to 15 atoms. Examples of cycloalkyl groups include but are not limited to cyclopropyl, cyclobutyl, cyclopentyl, cyclohexyl, cycloheptyl, cyclooctyl, cyclononyl, adamantanyl and cyclodecyl. An "optionally substituted cycloalkyl" refers to a cycloalkyl having optionally one or more substituents (for example 1 to 3 substituents, for example 1, 2, 3 or 4 substituents), selected from those defined above for substituted alkyl.

Where alkyl groups as defined are divalent, i.e., with two single bonds for attachment to two other groups, they are termed "alkylene" groups. Non-limiting examples of alkylene groups includes methylene, ethylene, methylmethylene, trimethylene, propylene, tetramethylene, ethylethylene, 1,2-dimethylethylene, pentamethylene and hexamethylene. Similarly, where alkenyl groups as defined above and alkynyl groups as defined above, respectively, are divalent radicals having single bonds for attachment to two other groups, they are termed "alkenylene" and "alkynylene" respectively.

The term "alkoxy" or "alkyloxy" as used herein refers to a radical having the Formula -OR‴ wherein R‴ is alkyl, alkenyl, or alkynyl. Non-limiting examples of suitable alkoxy include methoxy, ethoxy, propoxy, isopropoxy, butoxy, isobutoxy, sec-butoxy, tert-butoxy, pentyloxy and hexyloxy. The term "aryloxy" as used herein refers to a radical having the Formula -OR‴ wherein R‴ is aryl.

Where the oxygen atom in an alkoxy group is substituted with sulfur, the resultant radical is referred to as alkylthio or arylthio, such as methylthio, ethylthio, phenylthio, and the like.

The term "oxo" as used herein refers to the group =O.

The term "carbonyl" by itself or as part of another substituent refers to the group -C(O)R^{iv}, wherein R^{iv} is a hydrogen atom (i.e. an aldehyde), or alkyl, alkenyl, alkynyl or aryl (i.e. a ketone).

The term "carboxy" or "carboxyl" or "hydroxycarbonyl" by itself or as part of another substituent refers to the group -COOH, -C(O)OH, or -CO₂H.

The term "alkoxycarbonyl" by itself or as part of another substituent refers to a carboxy group linked to an alkyl radical i.e. to form -C(O)OR^{v}, wherein R^{v} is alkyl, alkenyl, alkynyl or aryl.

The term "alkylcarbonyloxy" by itself or as part of another substituent refers to a -OC(O)R^{vi} wherein R^{vi} is alkyl, alkenyl, alkynyl or aryl.

The term "heterocycle" as used herein by itself or as part of another group refers to non-aromatic, fully saturated or partially unsaturated cyclic groups (for example, 3 to 13 member monocyclic, 7 to 17 member bicyclic, or 10 to 20 member tricyclic ring systems, or containing a total of 3 to 10 ring atoms) which have at least one heteroatom in at least one carbon atom-containing ring. Each ring of the heterocyclic group containing a heteroatom may have 1, 2, 3 or 4 heteroatoms selected from nitrogen atoms, oxygen atoms and/or sulfur atoms, where the nitrogen and sulfur heteroatoms may optionally be oxidized and the nitrogen heteroatoms may optionally be quaternized. The heterocyclic group may be attached at any heteroatom or carbon atom of the ring or ring system, where valence allows. The rings of multi-ring heterocycles may be fused, bridged and/or joined through one or more spiro atoms. An optionally substituted heterocyclic refers to a heterocyclic having optionally one or more substituents (for example 1 to 4 substituents, or for example 1, 2, 3 or 4), selected from those defined above for substituted alkyl. Non-limiting examples of heterocycle comprise: piperidinyl, azepanyl, morpholinyl.

The term "aryl" as used herein refers to a polyunsaturated, aromatic hydrocarbyl group having a single ring (i.e. phenyl) or multiple aromatic rings fused together (e.g. naphthalene or anthracene) or linked covalently, typically containing 6 to 10 atoms; wherein at least one ring is aromatic. The aromatic ring may optionally include one to three additional rings (either cycloalkyl, heterocyclyl, or heteroaryl) fused thereto. Aryl is also intended to include the partially hydrogenated derivatives of the carbocyclic systems enumerated herein. Non-limiting examples of aryl comprise phenyl, napthyl, and the like. The aryl group or heterocycle as defined herein can optionally be substituted by one or more substituents (for example 1 to 5 substituents, for example 1, 2, 3, 4 or 5) at any available point of attachment. Non-limiting examples of such substituents are selected from halogen, hydroxyl, oxo, nitro, amino, hydrazine, aminocarbonyl, azido, cyano, alkyl, cycloalkyl, alkenyl, alkynyl, cycloalkylalkyl, alkylamino, alkoxy, -SO₂-NH₂, aryl, heteroaryl, aralkyl, haloalkyl, haloalkoxy, alkoxycarbonyl, alkylaminocarbonyl, heteroarylalkyl, alkylsulfonamide, heterocyclyl, alkylcarbonylaminoalkyl, aryloxy, alkylcarbonyl, acyl, arylcarbonyl, aminocarbonyl, alkylsulfoxide, -SO₂R^{v}, alkylthio, carboxyl, and the like, wherein R^{v} is alkyl or cycloalkyl.

The term "heteroaryl" as used herein by itself or as part of another group refers but is not limited to 5 to 12 carbon-atom aromatic rings or ring systems containing 1 to 3 rings which are fused together or linked covalently, typically containing 5 to 8 atoms; at least one of which is aromatic in which one or more carbon atoms in one or more of these rings can be replaced by oxygen, nitrogen or sulfur atoms where the nitrogen and sulfur heteroatoms may optionally be oxidized and the nitrogen heteroatoms may optionally be quaternized. Such rings may be fused to an aryl, cycloalkyl, heteroaryl or heterocyclyl ring. Non-limiting examples of such heteroaryl, include piridinyl, azepinyl.

An "optionally substituted heteroaryl" refers to a heteroaryl having optionally one or more substituents (for example 1 to 4 substituents, for example 1, 2, 3 or 4), selected from those defined above for substituted aryl.

As used herein the terms such as "alkyl, aryl, or cycloalkyl, each being optionally substituted with" or "alkyl, aryl, or cycloalkyl, optionally substituted with" refers to optionally substituted alkyl, optionally substituted aryl and optionally substituted cycloalkyl.

The term "halo" or "halogen" as a group or part of a group is generic for fluoro, chloro, bromo, or iodo.

The term "direct bond" as used herein, refers to a chemical linkage directly connecting two or more specified moieties, without the presence of any intervening elements or groups.

As used herein, when a compound has one or more stereocenters, each stereocenter may have the R or S configuration, unless stated otherwise. The compound may therefore be a racemic mixture of enantiomers and/or diastereoisomers, or it may have an excess of one or more of the enantiomers and/or diastereoisomers, such as more than 60 %, more than 70 %, more than 80 %, more than 85 %, more than 90 %, more than 95 %, more than 98 %, more than 99 %.

As already mentioned herein before, in a first aspect, the present invention provides a method for preparing a protected amine, the method comprising the steps of:
a) providing an organic carbonate A represented by formula (I) 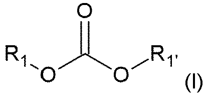 wherein
   R₁ and R_{1'} are each independently selected from any substituted or unsubstituted alkyl, any substituted or unsubstituted alkenyl, any substituted or unsubstituted cycloalkyl, and any substituted or unsubstituted aryl; or
   R₁ and R_{1'}, together with the -O-C-O- fragment to which they are attached, form one or more cyclic structures;
   with the proviso that R₁ and R_{1'} are not -CCl₃;
b) reacting, in a liquid medium, the organic carbonate A provided in step a) with an active hydrogen compound B, thereby obtaining a precursor C; wherein the active hydrogen compound B is selected from substituted or unsubstituted azoles, substituted or unsubstituted secondary amides, substituted or unsubstituted lactams, and substituted or unsubstituted oximes, preferably substituted or unsubstituted azoles, more preferably substituted or unsubstituted pyrazole; and
c) reacting the precursor C obtained in step b) with an amine D having one or more -NH₂ groups, thereby obtaining the protected amine.

The present invention provides for several advantages, and in particular, it provides for a method for preparing a protected amine, in particular a blocked isocyanate, without the use of phosgene or any of its analogues, such as diphosgene or triphosgene. The method according to the present invention rather uses organic carbonates as the one carbon (1C) synthon instead of phosgene or its analogues. Besides their rather benign characteristics compared to phosgene, organic carbonates have the additional advantage that they can be prepared from CO₂, which makes them a sustainable carbon source for chemical compounds. Further, the method as described herein avoids using free isocyanates in the process of preparing the protected amines, in particular the blocked isocyanates, and rather uses carbamic or carbonic esters as intermediates. The method as described herein uses mild reaction conditions which are tolerant to a variety of functional groups. The method as described herein also the advantage that the formation of by-products, either at step a) or step b), is minimal, thereby allowing higher yields of target products. Advantageously, the method according to the present inventions allows the formation of protected amines from amines comprising more than one single amine group, in particular a primary amine (-NH₂) moiety. In particular, the method as described herein allows for the formation of protected amines from a variety of amines, such as, and not limited to monoamines, diamines, triamines, tetramines, and other polyamines, as well as amines comprising further functional groups, such as amino acids, and amines comprising hydroxy (-OH) groups.

The method as described herein may be represented by the following reaction scheme, wherein an organic carbonate A is reacted with an active hydrogen compound B to form a precursor C, and wherein the precursor C is further reacted with an amine D to form the protected amine. The active hydrogen compound B is represented as H-B* to illustrate that the hydrogen atom of the active hydrogen compound B is removed during the substitution reaction of at least one of the alkoxy or aryloxy groups of the organic carbonate A, resulting in at least one fragment B* connected to the carbonyl group originating from the organic carbonate A. The precursor C can be a mono-product, wherein only one alkoxy or aryloxy group of the organic carbonate A is replaced by a fragment B*, a bis-product, wherein both alkoxy and/or aryloxy groups of the organic carbonate A are replaced by a fragment B*, or a mixture of both. The amine D is represented as a mono-amine in the upper right corner, thereby providing a protected mono-amine, whereas in the lower right corner, the amine D is represented as a diamine, thereby providing a protected diamine.

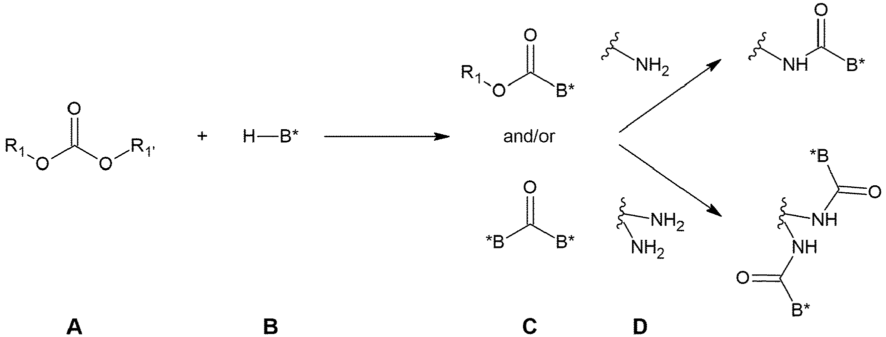

In particular, it has been observed that the use of lactams or oximes as active hydrogen compounds may provide bis-products of the precursor C.

In the context of the present invention, the terms 'protected amine' or 'protected amines' therefore refer to (a) chemical compound(s) comprising at least one protected amine moiety represented by formula (A) 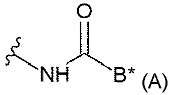 wherein
B* is a fragment of an active hydrogen compound B.

In the method according to the present invention, any organic carbonate A may be used which is capable of reacting with an active hydrogen compound B. The organic carbonate A may be represented by formula (I), wherein R₁ and R_{1'} are each independently selected from any substituted or unsubstituted alkyl, any substituted or unsubstituted alkenyl, any substituted or unsubstituted cycloalkyl, and any substituted or unsubstituted aryl, or R₁ and R_{1'}, together with the -O-C-O- fragment to which they are attached, form one or more cyclic structures. The organic carbonate A, however, cannot be triphosgene. The use of triphosgene does not provide the advantages of the present invention in comparison to phosgene based processes, as already described hereinbefore. Suitable examples of the organic carbonate A include diphenyl carbonate, dimethyl carbonate, diethyl carbonate, ethylene carbonate, propylene carbonate, and trimethylene carbonate.

In an embodiment, the present invention provides the method as defined herein, wherein the organic carbonate A provided in step a) is represented by formula (I) 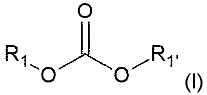 wherein
R₁ and R_{1'} are each independently selected from -C₁₋₁₂alkyl, -C₂₋₁₂alkenyl, -C₃₋₇cycloalkyl, and -Ar₁; wherein said -C₁₋₁₂alkyl, -C₂₋₁₂alkenyl, and -C₃₋₇cycloalkyl optionally comprises one or more heteroatoms selected from N, O, and S and/or is substituted with one or more substituents selected from -OH, -SH, and -NRₐRₐ';
or R₁ and R_{1'}, together with the -O-C(O)-O- fragment to which they are attached, form a 5-to 7-membered cyclic group; wherein said cyclic group is optionally fused to an aromatic or non-aromatic cycle and/or is substituted with one or more substituents selected from -C₁₋₆alkyl, -C₂₋₆alkenyl, -C₃₋₇cycloalkyl, -OH, -SH, and -NRₐRₐ';
each instance of Ar₁ is independently a 5- to 10-membered aromatic cycle optionally comprising one or more heteroatoms selected from O, N and S and/or substituted with one or more substituents independently selected from -halogen, -C₁₋₆alkyl, -C₂₋₆alkenyl, -C₃₋₇cycloalkyl, -OH, -SH, NRₐR_{a'}, and -OC₁₋₆alkyl; and
each instance of Rₐ and each instance of Rₐ is independently selected from -H, and -C₁₋₆alkyl.

In a particular embodiment, the present invention provides the method as defined herein, wherein the organic carbonate A provided in step a) is selected from diphenyl carbonate, dimethyl carbonate, diethyl carbonate, ethylene carbonate, propylene carbonate, and trimethylene carbonate.

It was found that diphenyl carbonate is particularly suited to be used in the method according to the invention. When diphenyl carbonate is used in step b), it may provide for high yields while facilitating the separation of precursor C during or after step b). The use of diphenyl carbonate may further provide for good selectivity, also at relatively low temperatures. Diphenyl carbonate can moreover be easily obtained from CO₂. When diphenyl carbonate is used in step b), the phenol by-product can be easily recycled (e.g. by solvent extraction, sublimation, distillation), which may further limit the carbon footprint of the method of the present invention. In a preferred embodiment, the present invention therefore provides the method as defined herein, wherein the organic carbonate A provided in step a) is diphenyl carbonate.

It was further found that particular active hydrogen compounds provide protected amines with improved properties.

When substituted or unsubstituted azoles, substituted or unsubstituted secondary amides, substituted or unsubstituted lactams, or substituted or unsubstituted oximes, are used as the active hydrogen compound B, the resulting protected amines may be unblocked to provide the corresponding isocyanates at lower temperatures, relative to protected amines wherein B* is - OPh or-NHPh.

In the context of the present invention, the term 'unblocking' refers to a reaction wherein the fragment B*, in this case also referred to as 'blocking group', is removed from the carbonyl group to which it is attached, to provide the corresponding isocyanate. The process of unblocking may be represented by the following reaction scheme.

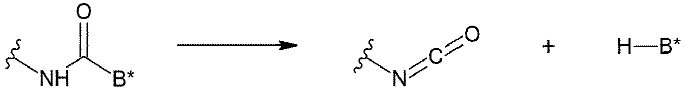

The protected amines according to the present invention may be unblocked by a variety of forms of energy, thereby providing the corresponding free isocyanate. In particular, the blocked isocyanates according to the present invention may be unblocked by providing heat, by reaction with an additive, or any combination thereof.

In an embodiment, the present invention provides the method as defined herein, wherein the active hydrogen compound B is selected from substituted or unsubstituted azoles.

When substituted or unsubstituted pyrazole is used as the active hydrogen compound B, the resulting protected amines may be advantageously deprotected to the corresponding amine by activating the pyrazole protecting group with a source of fluoride or hydroxide soluble in organic media, and subsequent contact with an aqueous solution.

In the context of the present invention, the term 'deprotecting' refers to a reaction wherein the fragment B*, as well as the carbonyl group to which it is attached, are removed to provide the corresponding amine.

The process of deprotecting may be represented by the following reaction scheme.

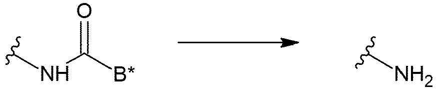

In a particular embodiment, the present invention provides the method as defined herein, wherein the active hydrogen compound B is selected from substituted or unsubstituted pyrazole.

In another particular embodiment, the present invention provides the method as defined herein, wherein the active hydrogen compound B is represented by formula (II) 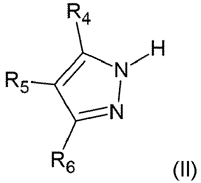 wherein
R₄, and R₆ are each independently selected from hydrogen, any halogen, any substituted or unsubstituted alkyl, any substituted or unsubstituted alkenyl, any substituted or unsubstituted cycloalkyl, and any substituted or unsubstituted aryl; and
R₅ is selected from hydrogen, hydroxy, thio, any substituted or unsubstituted amino, any substituted or unsubstituted alkoxy or aryloxy, any substituted or unsubstituted alkyl, any substituted or unsubstituted alkenyl, any substituted or unsubstituted cycloalkyl, any substituted or unsubstituted aryl, any substituted or unsubstituted ester, any substituted or unsubstituted carbonyl, any substituted or unsubstituted amide, any substituted or unsubstituted carbonate, and any substituted or unsubstituted carbamate.

In a further particular embodiment, the present invention provides the method as defined herein, wherein the active hydrogen compound B is represented by formula (II) 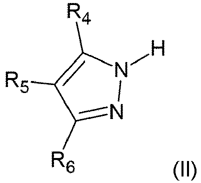 wherein
R₄ and R₆ are each independently selected from -H, -halogen, -C₁₋₁₂alkyl, -C₂₋₁₂alkenyl, -C₃₋₇cycloalkyl, and Ar₄; wherein said -C₁₋₁₂alkyl, -C₂₋₁₂alkenyl, and -C₃₋₇cycloalkyl optionally comprises one or more heteroatoms selected from N, O, and S and/or is substituted with one or more substituents selected from -halogen;
R₅ is selected from -H, -OH, -SH, -NR_{g}R_{g'}, -C₁₋₁₂alkyl, -C₂₋₁₂alkenyl, -C₃₋₇cycloalkyl, -CHO, - C(O)Rₕ, -COOH, -OC(O)Rₕ, -C(O)ORₕ, -OC(O)ORₕ, -NR_{g}C(O)Rₕ, -C(O)NR_{g}R_{g'}, - NR_{g}C(O)ORₕ, and -OC(O)NR_{g}R_{g'}-, and Ar₅; wherein said -C₁₋₁₂alkyl, -C₂₋₁₂alkenyl, and -C₃₋₇cycloalkyl optionally comprises one or more heteroatoms selected from N, O, and S and/or is substituted with one or more substituents selected from -H, -OH, -SH, -NRᵢR_{i'}, -C₁₋₁₂alkyl, -C₂₋₁₂alkenyl, -C₃₋₇cycloalkyl, -CHO, -C(O)Rⱼ, -COOH, -OC(O)Rⱼ, -C(O)ORⱼ, -OC(O)ORⱼ, - NRᵢC(O)Rⱼ, -C(O)NRᵢR_{i'}, -NRᵢC(O)ORⱼ, -OC(O)NRᵢR_{i'}-, and Ar₆;
Ar₄, Ar₅, and Ar₆, are each independently a 5- to 10-membered aromatic cycle optionally comprising one or more heteroatoms selected from O, N and S and/or substituted with one or more substituents independently selected from -halogen, -C₁₋₆alkyl, -C₂₋₆alkenyl, -C₃₋₇cycloalkyl, -OH, -SH, NR_{f}R_{f'}, and -OC₁₋₆alkyl;
each instance of R_{g}, each instance of R_{g}, and each instance of Rₕ, each instance of Rᵢ, each instance of R_{i'} and each instance of Rⱼ is independently selected from -H, and -C₁₋₁₂alkyl.

In a further particular embodiment, the present invention provides the method as defined herein, wherein the active hydrogen compound B is represented by formula (II) 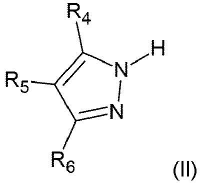 wherein
R₄ and R₆ are each -CH₃; and
R₅ is -H.

In the context of the present invention, the term 'liquid medium' refers to a medium in a liquid state capable of solubilizing and/or dispersing one or more reactants.

It was found that some organic carbonates, such as diphenyl carbonate, can be used as a liquid medium in step b) at a reaction temperature equal to or above their melting point. This can avoid the use of any additional solvents, which may lead to a more environmentally friendly method. It was also found that the use of one or more solvents may be advantageous in that step b) may be performed at lower temperatures, even at room temperature, with satisfactory yields. This may be particularly advantageous when the reaction is performed at a temperature below the melting temperature of the organic carbonate A. In an embodiment, the present invention therefore provides the method as defined herein, wherein the liquid medium in step b) is the organic carbonate A, one or more solvents, or any combination thereof.

The method of the present invention is not limited to any solvents. It may however be preferred to use solvents that are not prone to reacting with any of the reactants used in step b) and/or step c) at the reaction temperature. Suitable examples include THF, 2-MeTHF, MTBE, dichloromethane, acetonitrile, acetone, ethyl acetate, DMF, toluene, xylene, and anisole.

While step b) may be performed at any reaction temperature wherein the carbonate A reacts with the active hydrogen compound B, lower reaction temperatures may be advantageous in avoiding decomposition of the reactants and/or any other side-reactions occurring, in particular when heat sensitive reactants are used, thereby avoiding more laborious work-up. As already mentioned hereinbefore, the presence of a solvent may be advantageous and/or necessary to perform the reaction at a lower reaction temperature, in particular at a reaction temperature below the melting temperature of the organic carbonate A. In an embodiment, the present invention provides the method as defined herein, wherein in step b), the reaction temperature is at least 50 °C, preferably at least 60 °C, more preferably from 70 to 120 °C, even more preferably from 80 to 100 °C.

While step c) may be performed at any reaction temperature wherein the precursor C reacts with the amine D, lower reaction temperatures may be advantageous in avoiding decomposition of the reactants and/or any other side-reactions occurring, in particular when heat sensitive reactants are used, thereby avoiding more laborious work-up. As already mentioned hereinbefore, the presence of a solvent may be advantageous and/or necessary to perform the reaction at a lower reaction temperature. In an embodiment, the present invention provides the method as defined herein, wherein in step b), the reaction temperature is at most 60 °C, preferably at most 50 °C, more preferably from 0 to 40 °C, even more preferably from 10 to 30 °C.

It appeared that the presence of a nucleophilic catalyst may be advantageous when the organic carbonate A is reacted with the active hydrogen compound B in step b), as this may lead to a faster reaction, a lower reaction temperature, a higher selectivity and/or improved yields of the precursor C. In an embodiment, the present invention therefore provides the method as defined herein, wherein step b) is performed in the presence of a nucleophilic catalyst.

While the method of the present invention is not limited to particular nucleophilic catalysts, it was found that strong bases, such as sodium hydroxide (NaOH), sodium methoxide, sodium tert-butoxide and potassium hydroxide (KOH) performed inferior in catalysing the reaction of the organic carbonate A with the active hydrogen compound B in step b), relative to other bases, such as 4-dimethylaminopyridine (DMAP), 1,8-diazabicyclo[5.4.0]undec-7-ene (DBU), 1,5,7-triazabicyclo[4.4.0]dec-5-ene (TBD), 1,5-diazabicyclo[4.3.0]non-5-ene (DBN), imidazole, N-methylimidazole, *p*-methoxypyridine *N*-oxide (MPO), hexamethylenetetramine (HMTA), 1,4-diazabicyclo[2.2.2]octane (DABCO), pyridine, lutidine, triethylamine (TEA), triphenylphosphine (TPP), 4-pyrrolidinopyridine, N-methylmorpholine, pyrazine, and pyrimidine. In a preferred embodiment, the present invention therefore provides the method as defined herein, wherein the nucleophilic catalyst is selected from 4-dimethylaminopyridine (DMAP), 1,8-diazabicyclo[5.4.0]undec-7-ene (DBU), 1,5,7-triazabicyclo[4.4.0]dec-5-ene (TBD), 1,5-diazabicyclo[4.3.0]non-5-ene (DBN), N-methylimidazole, hexamethylenetetramine (HMTA), 1,4-diazabicyclo[2.2.2]octane (DABCO), triethylamine (TEA), 4-pyrrolidinopyridine, and N-methylmorpholine, in particular 4-dimethylaminopyridine (DMAP).

In a particular embodiment, the present invention provides the method as defined herein, wherein the nucleophilic catalyst is present in step b) in an amount of at least 0.01 molar equivalent, relative to the amount of organic carbonate A, preferably from 0.01 to 1.0 molar equiv., more preferably from 0.01 to 0.50 molar equiv., even more preferably in an amount from 0.02 to 0.20 molar equiv., yet even more preferably in an amount from 0.05 to 0.10 molar equiv. It was found particularly advantageous to provide the nucleophilic catalyst at these concentrations, as higher yields of the precursor C may be obtained.

In an embodiment, the present invention provides the method as defined herein, wherein the molar ratio of the organic carbonate A relative to the active hydrogen compound B, is from 1.0:0.25 to 1.0:4.0, preferably from 1.0:1.0 to 1.0:4.0, more preferably from 1.0:1.5 to 1.0:2.5.

It further appeared to be advantageous to separate the protected precursor C obtained in step b) from one or more by-products and/or unreacted reactants, before it is reacted in step c) with the amine D, as this may provide for a less laborious work-up to obtain the protected amine D and/or a better yield of the protected amine D. It moreover may allow to recycle any unreacted reactants and/or any by-products. This additional separation step may be performed by solvent extraction, sublimation and/or distillation. In an embodiment, the present invention provides the method as defined herein, further comprising the step of separating the protected precursor obtained in step b) from one or more by-products and/or unreacted reactants by solvent extraction, sublimation and/or distillation of said by-products and/or unreacted reactants, before it is reacted in step c) with the amine D.

In the method of the present invention, the amine D comprises at least one -NH₂ moiety. Amine D may contain one, two, three, or more -NH₂ moieties, also referred to as monoamines, diamines, triamines, and polyamines, respectively.

Examples of amine D according to embodiments of the present invention include ethylenediamine, propylenediamine, tetramethylenediamine, pentamethylenediamine, hexamethylenediamine, isophorone diamine, 4,4'-diaminodicyclohexylmethane, 4,4'-diaminodiphenylmethane, and 2,4-diaminotoluene.

In an embodiment, the present invention provides the method as defined herein, wherein the amine D comprises at least two -NH₂ moieties. When amine D comprises at least two -NH₂ moieties, the resulting protected amine can be unblocked to the corresponding polyisocyanate, in particular diisocyanate, which can be used in the synthesis of polymers, such as polyurethanes and polyureas.

The amine D optionally comprises one or more functional groups other than primary amine (-NH₂) groups, such as secondary or tertiary amine groups, hydroxy (-OH) groups, alkoxy or aryloxy groups (-OR‴), carboxylic acid (-COOH) groups, ester groups (-C(O)ORᵥ), nitrile (-CN) groups, unsaturated bonds, halogens. As mentioned hereinbefore, it is an advantage of the method of the invention that it has a high tolerance towards different functional groups.

In another embodiment, the present invention provides the method as defined herein, wherein the amine D is an alkanolamine. In the context of the present invention, the term 'alkanolamine' refers to a chemical compound that comprises at least one primary amine (-NH₂) group and at least one hydroxy (-OH) group.

Further examples of amine D according to embodiments of the present invention include 2-aminoethanol, 1-amino-2-propanol, and 6-aminohexan-1-ol.

In another embodiment, the present invention provides the method as defined herein, wherein the amine D is an amino acid or an amino acid ester. Amine D can be any amino acid or amino acid ester, such as an alpha-, beta-, gamma- or delta- amino acid or amino acid ester.

The amino acid can be commercially available, or it can for instance be prepared by fermentation or by a chemical method, such as a Strecker reaction. An amino acid obtained by fermentation often has an L-configuration as its main component, while an amino acid obtained by a chemical method is often a racemic mixture of the D- and L-configuration. The amino acid may be purified into a form of a hydrochloride (-NH₂•HCl), or it may have an amino group (-NH₂) free from hydrochloric acid. Preferably the amino acid has a purity of 90% or more. The purity is the ratio of the mass of the amino acid, relative to the total mass. The amino acid has more preferably a purity of 97% or more. The purity of the amino acid may be 99.5% or less, or 98% or less.

Suitable examples of amine D according to embodiments of the present invention include Proteinogenic amino acids, such as L-Alanine (Ala), L-Arginine (Arg), L-Asparagine (Asn), L-Aspartic acid (Asp), L-Cysteine (Cys), L-Glutamic acid (Glu), L-Glutamine (Gln), Glycine (Gly), L-Histidine (His), L-lsoleucine (Ile), L-Leucine (Leu), L-Lysine (Lys), L-Methionine (Met), L-Phenylalanine (Phe), L-Serine (Ser), L-Threonine (Thr), L-Tryptophan (Trp), L-Tyrosine (Tyr), L-Valine (Val), L-Selenocysteine (Sec), and L-Pyrrolysine (Pyl); D-amino acids; homo amino acids; beta-homo amino acids; alpha-methyl amino acids; non-natural side chain variant amino acids and other unusual amino acids, such as citrulline (Cit), norleucine (Nle), 3-nitrotyrosine, nitroarginine, ornithine (Orn), naphtylalanine (Nal), Abu, DAB, methionine sulfoxide or methionine sulfone.

In a preferred embodiment, the present invention provides the method as defined herein, wherein the amine D is an alpha-amino acid or an alpha-amino acid ester. The alpha-amino acid or an alpha-amino acid ester used in step c) of the method as described herein can be any naturally occurring alpha-amino acid or an alpha-amino acid ester, or any non-naturally occurring alpha-amino acid or an alpha-amino acid ester. The alpha-amino acid or an alpha-amino acid ester used in step c) of the method as described herein, can have either a L- or D-configuration, or it can be provided as a mixture of both configurations. Preferably, the alpha-amino acid or an alpha-amino acid ester used in step c) of the method as described herein has an L-configuration.

When amine D is an alpha-amino acid or an alpha-amino acid ester, the resulting protected amine can be used in the preparation of oligopeptides or polypeptides. When amine D is an alpha-amino acid or an alpha-amino acid ester, the resulting protected amine can alternatively be unblocked to provide an amino acid or amino acid ester bearing an isocyanate moiety, which can be further functionalized to provide oligopeptide analogues comprising a carbamate or urea moiety.

In a particular embodiment, the present invention provides the method as defined herein, wherein the amine D is an alpha-amino acid selected from Alanine (Ala), Arginine (Arg), Asparagine (Asn), Aspartic acid (Asp), Cysteine (Cys), Glutamic acid (Glu), Glutamine (Gln), Glycine (Gly), Histidine (His), Isoleucine (Ile), Leucine (Leu), Lysine (Lys), Methionine (Met), Phenylalanine (Phe), Serine (Ser), Threonine (Thr), Tryptophan (Trp), Tyrosine (Tyr), Valine (Val), Selenocysteine (Sec), Pyrrolysine (Pyl), or any of their corresponding alpha-amino acid esters.

In a particular embodiment, the present invention provides the method as defined herein, wherein the amine D is represented by formula (III) 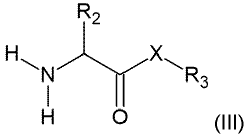 wherein
R₂ is selected from -H, -OH, -SH, SeH, -NR_{b}R_{b}, -C₁₋₁₂alkyl, -C₂₋₁₂alkenyl, -C₃₋₇cycloalkyl, - CHO, -C(O)R_{c}, -COOH, -OC(O)R_{c}, -C(O)OR_{c}, -OC(O)OR_{c}, -NR_{b}C(O)R_{c}, -C(O)NR_{b}R_{b}, - NR_{b}C(O)OR_{c}, and -OC(O)NR_{b}R_{b'}-, and Ar₂; wherein said -C₁₋₁₂alkyl, -C₂₋₁₂alkenyl, and -C₃₋₇cycloalkyl optionally comprises one or more heteroatoms selected from N, O, and S and/or is substituted with one or more substituents selected from -H, -OH, -SH, -NR_{d}R_{d'}, -C₁₋₁₂alkyl, -C₂₋₁₂alkenyl, -C₃₋₇cycloalkyl, -CHO, -C(O)Rₑ, -COOH, -OC(O)Rₑ, -C(O)ORₑ, -OC(O)ORₑ, - NR_{d}C(O)Rₑ, -C(O)NR_{d}R_{d'}, -NR_{d}C(O)ORₑ, -OC(O)NR_{d}R_{d'}-, and Ar₃;
each instance of R_{b}, each instance of R_{b'}, each instance of R_{c}, each instance of R_{d}, each instance of R_{d'} and each instance of Rₑ is independently selected from -H, and -C₁₋₁₂alkyl; Ar₂ and Ar₃ are each independently a 5- to 10-membered aromatic cycle optionally comprising one or more heteroatoms selected from O, N and S and/or substituted with one or more substituents independently selected from -halogen, -C₁₋₆alkyl, -C₂₋₆alkenyl, -C₃₋₇cycloalkyl, -OH, -SH, NR_{f}R_{f'}, and -OC₁₋₆alkyl;
each instance of R_{f} and each instance of R_{f'} is independently selected from -H, and -C₁₋₆alkyl;
X is selected from -O-, and -NH-; and
R₃ is selected from -H, and -C₁₋₁₂alkyl, or R₃ is a solid support.

In another particular embodiment, the present invention provides the method as defined herein, wherein the amine D is represented by formula (III), wherein R₃ is -H.

In yet another particular embodiment, the present invention provides the method as defined herein, wherein the amine D is represented by formula (Illa) 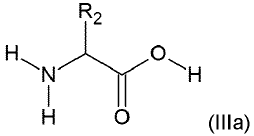 wherein
R₂ is selected from -H, -OH, -SH, SeH, -NR_{b}R_{b}, -C₁₋₁₂alkyl, -C₂₋₁₂alkenyl, -C₃₋₇cycloalkyl, - CHO, -C(O)R_{c}, -COOH, -OC(O)R_{c}, -C(O)OR_{c}, -OC(O)OR_{c}, -NR_{b}C(O)R_{c}, -C(O)NR_{b}R_{b}, - NR_{b}C(O)OR_{c}, and -OC(O)NR_{b}R_{b'}-, and Ar₂; wherein said -C₁₋₁₂alkyl, -C₂₋₁₂alkenyl, and -C₃₋₇cycloalkyl optionally comprises one or more heteroatoms selected from N, O, and S and/or is substituted with one or more substituents selected from -H, -OH, -SH, -NR_{d}R_{d'}, -C₁₋₁₂alkyl, -C₂₋₁₂alkenyl, -C₃₋₇cycloalkyl, -CHO, -C(O)Rₑ, -COOH, -OC(O)Rₑ, -C(O)ORₑ, -OC(O)ORₑ, - NR_{d}C(O)Rₑ, -C(O)NR_{d}R_{d'}, -NR_{d}C(O)ORₑ, -OC(O)NR_{d}R_{d'}-, and Ar₃;
each instance of R_{b}, each instance of R_{b'}, each instance of R_{c}, each instance of R_{d}, each instance of R_{d'} and each instance of Rₑ is independently selected from -H, and -C₁₋₁₂alkyl; Ar₂ and Ar₃ are each independently a 5- to 10-membered aromatic cycle optionally comprising one or more heteroatoms selected from O, N and S and/or substituted with one or more substituents independently selected from -halogen, -C₁₋₆alkyl, -C₂₋₆alkenyl, -C₃₋₇cycloalkyl, -OH, -SH, NR_{f}R_{f'}, and -OC₁₋₆alkyl; and
each instance of R_{f} and each instance of R_{f'} is independently selected from -H, and -C₁₋₆alkyl.

According to a second aspect, the present invention provides a protected amine obtainable by the method as defined herein.

According to a third aspect, the present invention provides a protected amine, in particular a protected alpha-amino acid or an alpha-amino acid ester, represented by formula (IV) 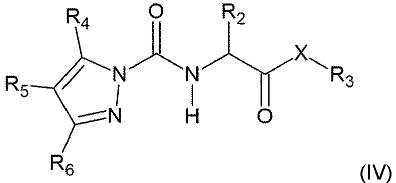 wherein
R₂ is selected from -H, -OH, -SH, SeH, -NR_{b}R_{b}, -C₁₋₁₂alkyl, -C₂₋₁₂alkenyl, -C₃₋₇cycloalkyl, - CHO, -C(O)R_{c}, -COOH, -OC(O)R_{c}, -C(O)OR_{c}, -OC(O)OR_{c}, -NR_{b}C(O)R_{c}, -C(O)NR_{b}R_{b}, - NR_{b}C(O)OR_{c}, and -OC(O)NR_{b}R_{b'}-, and Ar₂; wherein said -C₁₋₁₂alkyl, -C₂₋₁₂alkenyl, and -C₃₋₇cycloalkyl optionally comprises one or more heteroatoms selected from N, O, and S and/or is substituted with one or more substituents selected from -H, -OH, -SH, -NR_{d}R_{d'}, -C₁₋₁₂alkyl, -C₂₋₁₂alkenyl, -C₃₋₇cycloalkyl, -CHO, -C(O)Rₑ, -COOH, -OC(O)Rₑ, -C(O)ORₑ, -OC(O)ORₑ, - NR_{d}C(O)Rₑ, -C(O)NR_{d}R_{d'}, -NR_{d}C(O)ORₑ, -OC(O)NR_{d}R_{d'}-, and Ar₃;
each instance of R_{b}, each instance of R_{b'}, each instance of R_{c}, each instance of R_{d}, each instance of R_{d'} and each instance of Rₑ is independently selected from -H, and -C₁₋₁₂alkyl; Ar₂ and Ar₃ are each independently a 5- to 10-membered aromatic cycle optionally comprising one or more heteroatoms selected from O, N and S and/or substituted with one or more substituents independently selected from -halogen, -C₁₋₆alkyl, -C₂₋₆alkenyl, -C₃₋₇cycloalkyl, -OH, -SH, NR_{f}R_{f'}, and -OC₁₋₆alkyl;
each instance of R_{f} and each instance of R_{f'} is independently selected from -H, and -C₁₋₆alkyl;
X is selected from -O-, and -NH-;
R₃ is selected from -H, and -C₁₋₁₂alkyl, or R₃ is a solid support;
R₄, and R₆ are each independently selected from hydrogen, any halogen, any substituted or unsubstituted alkyl, any substituted or unsubstituted alkenyl, any substituted or unsubstituted cycloalkyl, and any substituted or unsubstituted aryl; and
R₅ is selected from hydrogen, hydroxy, thio, any substituted or unsubstituted amino, any substituted or unsubstituted alkoxy or aryloxy, any substituted or unsubstituted alkyl, any substituted or unsubstituted alkenyl, any substituted or unsubstituted cycloalkyl, any substituted or unsubstituted aryl, any substituted or unsubstituted ester, any substituted or unsubstituted carbonyl, any substituted or unsubstituted amide, any substituted or unsubstituted carbonate, and any substituted or unsubstituted carbamate.

As already mentioned hereinbefore, protected alpha-amino acids or alpha-amino acid esters obtainable by the method as described herein can be used in the preparation of oligopeptides or polypeptides. The particular alpha-amino acids or alpha-amino acid esters, protected with a substituted or unsubstituted pyrazolocarbonyl moiety, as defined herein, were found to provide several advantages.

The pyrazolocarbonyl protecting group is stable under both acidic and mild alkaline conditions, thereby allowing to add further amino acid residues to the C-terminus of the amino acid (or oligopeptide) under different conditions, while maintaining protection of the N-terminus. Once all the desired amino acid residues are added to provide the envisaged protected oligopeptide or protected polypeptide, the pyrazolocarbonyl protecting group can be easily and advantageously deprotected to the corresponding amine by activation of the protecting group, for instance with a source of fluoride or hydroxide soluble in organic media, and reaction with a nucleophile, such as an aqueous solution.

The pyrazolocarbonyl protecting group may also be advantageously deprotected to the corresponding alpha-amino acid or alpha-amino acid ester, which can be coupled with a further protected alpha-amino acid or alpha-amino acid ester via the N-terminus. This series of steps may be repeated to add further amino acid residues. Once all the desired amino acid residues are added to provide the envisaged protected oligopeptide or protected polypeptide, the pyrazolocarbonyl protecting group can be again be easily and advantageously deprotected to the corresponding amine.

In an embodiment, the present invention provides the protected amine as defined herein, and represented by formula (IV) 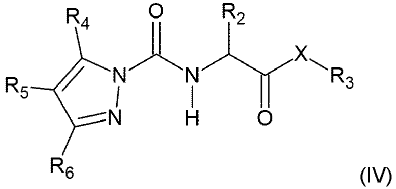 wherein
R₂ is selected from -H, -OH, -SH, SeH, -NR_{b}R_{b}, -C₁₋₁₂alkyl, -C₂₋₁₂alkenyl, -C₃₋₇cycloalkyl, - CHO, -C(O)R_{c}, -COOH, -OC(O)R_{c}, -C(O)OR_{c}, -OC(O)OR_{c}, -NR_{b}C(O)R_{c}, -C(O)NR_{b}R_{b}, - NR_{b}C(O)OR_{c}, and -OC(O)NR_{b}R_{b'}-, and Ar₂; wherein said -C₁₋₁₂alkyl, -C₂₋₁₂alkenyl, and -C₃₋₇cycloalkyl optionally comprises one or more heteroatoms selected from N, O, and S and/or is substituted with one or more substituents selected from -H, -OH, -SH, -NR_{d}R_{d'}, -C₁₋₁₂alkyl, -C₂₋₁₂alkenyl, -C₃₋₇cycloalkyl, -CHO, -C(O)Rₑ, -COOH, -OC(O)Rₑ, -C(O)ORₑ, -OC(O)ORₑ, - NR_{d}C(O)Rₑ, -C(O)NR_{d}R_{d'}, -NR_{d}C(O)ORₑ, -OC(O)NR_{d}R_{d'}-, and Ar₃;
X is selected from -O-, and -NH-;
R₃ is selected from -H, and -C₁₋₁₂alkyl, or R₃ is a solid support;
R₄ and R₆ are each independently selected from -H, -halogen, -C₁₋₁₂alkyl, -C₂₋₁₂alkenyl, -C₃₋₇cycloalkyl, and Ar₄; wherein said -C₁₋₁₂alkyl, -C₂₋₁₂alkenyl, and -C₃₋₇cycloalkyl optionally comprises one or more heteroatoms selected from N, O, and S and/or is substituted with one or more substituents selected from -halogen;
R₅ is selected from -H, -OH, -SH, -NR_{g}R_{g'}, -C₁₋₁₂alkyl, -C₂₋₁₂alkenyl, -C₃₋₇cycloalkyl, -CHO, - C(O)Rₕ, -COOH, -OC(O)Rₕ, -C(O)ORₕ, -OC(O)ORₕ, -NR_{g}C(O)Rₕ, -C(O)NR_{g}R_{g'}, - NR_{g}C(O)ORₕ, and -OC(O)NR_{g}R_{g'}-, and Ar₅; wherein said -C₁₋₁₂alkyl, -C₂₋₁₂alkenyl, and -C₃₋₇cycloalkyl optionally comprises one or more heteroatoms selected from N, O, and S and/or is substituted with one or more substituents selected from -H, -OH, -SH, -NRᵢR_{i'}, -C₁₋₁₂alkyl, -C₂₋₁₂alkenyl, -C₃₋₇cycloalkyl, -CHO, -C(O)Rⱼ, -COOH, -OC(O)Rⱼ, -C(O)ORⱼ, -OC(O)ORⱼ, - NRᵢC(O)Rⱼ, -C(O)NRᵢR_{i'}, -NRᵢC(O)ORⱼ, -OC(O)NRᵢR_{i'}-, and Ar₆;
Ar₂, Ar₃ Ar₄, Ar₅, and Ar₆ are each independently a 5- to 10-membered aromatic cycle optionally comprising one or more heteroatoms selected from O, N and S and/or substituted with one or more substituents independently selected from -halogen, -C₁₋₆alkyl, -C₂₋₆alkenyl, -C₃₋₇cycloalkyl, -OH, -SH, NR_{f}R_{f'}, and -OC₁₋₆alkyl;
each instance of R_{b}, each instance of R_{b'}, each instance of R_{c}, each instance of R_{d}, each instance of R_{d'}, each instance of Rₑ, each instance of R_{g}, each instance of R_{g'}, each instance of Rₕ, each instance of Rᵢ, each instance of R_{i'} and each instance of Rⱼ is independently selected from -H, and -C₁₋₁₂alkyl; and
each instance of R_{f} and each instance of R_{f'} is independently selected from -H, and -C₁₋₆alkyl.

It was further found that the protected alpha-amino acids are preferred over alpha-amino acid esters, as this avoids the additional step of hydrolysing the ester moiety to the corresponding carboxylic acid moiety to enable coupling with an additional amino acid residue via its C-terminus. It was also found that the protected alpha-amino acids have an enhanced solubility in water, compared to the corresponding alpha-amino acid esters. It is an advantage of the method as described herein that protected alpha-amino acids can be directly obtained from the corresponding amino acids, in contrast to methods where phosgene or free isocyanates are used in the preparation method.

In an embodiment, the present invention provides the protected amine as defined herein, and represented by formula (IV), wherein R₃ is H.

In another embodiment, the present invention provides the protected amine as defined herein, and represented by formula (lVa) 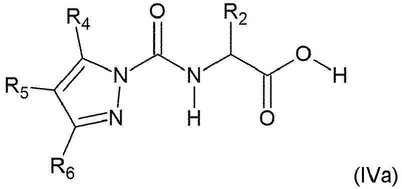 wherein
R₂ is selected from -H, -OH, -SH, SeH, -NR_{b}R_{b}, -C₁₋₁₂alkyl, -C₂₋₁₂alkenyl, -C₃₋₇cycloalkyl, - CHO, -C(O)R_{c}, -COOH, -OC(O)R_{c}, -C(O)OR_{c}, -OC(O)OR_{c}, -NR_{b}C(O)R_{c}, -C(O)NR_{b}R_{b}, - NR_{b}C(O)OR_{c}, and -OC(O)NR_{b}R_{b'}-, and Ar₂; wherein said -C₁₋₁₂alkyl, -C₂₋₁₂alkenyl, and -C₃₋₇cycloalkyl optionally comprises one or more heteroatoms selected from N, O, and S and/or is substituted with one or more substituents selected from -H, -OH, -SH, -NR_{d}R_{d'}, -C₁₋₁₂alkyl, -C₂₋₁₂alkenyl, -C₃₋₇cycloalkyl, -CHO, -C(O)Rₑ, -COOH, -OC(O)Rₑ, -C(O)ORₑ, -OC(O)ORₑ, - NR_{d}C(O)Rₑ, -C(O)NR_{d}R_{d'}, -NR_{d}C(O)ORₑ, -OC(O)NR_{d}R_{d'}-, and Ar₃;
R₄ and R₆ are each independently selected from -H, -halogen, -C₁₋₁₂alkyl, -C₂₋₁₂alkenyl, -C₃₋₇cycloalkyl, and Ar₄; wherein said -C₁₋₁₂alkyl, -C₂₋₁₂alkenyl, and -C₃₋₇cycloalkyl optionally comprises one or more heteroatoms selected from N, O, and S and/or is substituted with one or more substituents selected from -halogen;
R₅ is selected from -H, -OH, -SH, -NR_{g}R_{g'}, -C₁₋₁₂alkyl, -C₂₋₁₂alkenyl, -C₃₋₇cycloalkyl, -CHO, - C(O)Rₕ, -COOH, -OC(O)Rₕ, -C(O)ORₕ, -OC(O)ORₕ, -NR_{g}C(O)Rₕ, -C(O)NR_{g}R_{g'}, - NR_{g}C(O)ORₕ, and -OC(O)NR_{g}R_{g'}-, and Ar₅; wherein said -C₁₋₁₂alkyl, -C₂₋₁₂alkenyl, and -C₃₋₇cycloalkyl optionally comprises one or more heteroatoms selected from N, O, and S and/or is substituted with one or more substituents selected from -H, -OH, -SH, -NRᵢR_{i'}, -C₁₋₁₂alkyl, -C₂₋₁₂alkenyl, -C₃₋₇cycloalkyl, -CHO, -C(O)Rⱼ, -COOH, -OC(O)Rⱼ, -C(O)ORⱼ, -OC(O)ORⱼ, - NRᵢC(O)Rⱼ, -C(O)NRᵢR_{i'}, -NRᵢC(O)ORⱼ, -OC(O)NRᵢR_{i'}-, and Ar₆;
Ar₂, Ar₃ Ar₄, Ar₅, and Ar₆ are each independently a 5- to 10-membered aromatic cycle optionally comprising one or more heteroatoms selected from O, N and S and/or substituted with one or more substituents independently selected from -halogen, -C₁₋₆alkyl, -C₂₋₆alkenyl, -C₃₋₇cycloalkyl, -OH, -SH, NR_{f}R_{f'}, and -OC₁₋₆alkyl;
each instance of R_{b}, each instance of R_{b'}, each instance of R_{c}, each instance of R_{d}, each instance of R_{d'}, each instance of Rₑ, each instance of R_{g}, each instance of R_{g'}, each instance of Rₕ, each instance of Rᵢ, each instance of R_{i'} and each instance of Rⱼ is independently selected from -H, and -C₁₋₁₂alkyl; and
each instance of R_{f} and each instance of R_{f'} is independently selected from -H, and -C₁₋₆alkyl.

In an embodiment, the present invention provides the protected amine as defined herein, and represented by formula (IV) or (lVa), wherein R₄ and R₆ are each -CH₃; and R₅ is -H.

According to a fourth aspect, the present invention provides a method for preparing a protected dipeptide, the method comprising the steps of:
a) providing a protected amine, in particular a protected alpha-amino acid, as defined herein; and
b) coupling the protected amine provided in step a) with a further alpha-amino acid compound, thereby obtaining the protected dipeptide.

In the context of the present invention, and unless stated otherwise, the terms 'protected dipeptide', 'protected oligopeptide' or 'protected polypeptide' refer to the corresponding dipeptide, oligopeptide or polypeptide, respectively, wherein the N-terminus is protected with a pyrazolocarbonyl protecting group, as described herein.

As mentioned hereinbefore, when the protected amine is a protected alpha-amino acid, it can be directly coupled with a further amino acid. If the protected amine is a protected alpha-amino acid ester, the ester moiety needs to be hydrolysed first to enable coupling.

In an embodiment, the present invention provides a method for preparing a protected dipeptide as defined herein, wherein the protected amine provided in step a) is an alpha-amino acid.

In a specific embodiment, the present invention provides a method for preparing a protected dipeptide, the method comprising the steps of:
a) providing a protected alpha-amino acid, as defined herein; and
b) coupling the protected alpha-amino acid provided in step a) with a further alpha-amino acid compound, thereby obtaining the protected dipeptide.

In another embodiment, the present invention provides a method for preparing a protected dipeptide as defined herein, wherein the protected amine provided in step a) is an alpha-amino acid ester, the method comprising the further step of hydrolysing the ester moiety to provide the corresponding protected alpha-amino acid, and coupling the alpha-amino acid with the further alpha-amino acid compound in step b).

In a specific embodiment, the present invention provides a method for preparing a protected dipeptide, the method comprising the steps of:
a1) providing a protected alpha-amino ester, as defined herein;
a2) hydrolysing the ester moiety to provide the corresponding protected alpha-amino acid; and
b) coupling the protected alpha-amino acid provided in step a2) with a further alpha-amino acid compound, thereby obtaining the protected dipeptide.

The further alpha-amino acid compound used in step b) can be an alpha-amino acid ester or an alpha-amino acid bearing a carboxylic acid protecting group on its C-terminus. Carboxylic acid protecting groups, including esters, are known in the art, and include tert-butyl (t-Bu), benzyl (Bn), 2-chlorotrityl (2-Cl-Trt), 2,4-dimethoxybenzyl (Dmb), 2-phenylisopropyl(2-PhiPr), 5-phenyl-3,4-ethylenedioxythenyl derivatives (Phenyl-EDOTn), 9-fluorenylmethyl (Fm), 4-(N-[1-(4,4-dimethyl-2,6-dioxocyclohexylidene)-3-methylbutyl]-amino)benzyl (Dmab), methyl (Me), ethyl (Et), carbamoylmethyl (Cam), allyl (Al), benzyl (Bn), phenacyl (Pac), 2-trimethylsilylethyl, a metal cation.

The further alpha-amino acid compound used in step b) can also be an alpha-amino acid residue connected to a solid support. When the further alpha-amino acid compound used in step b) is an alpha-amino acid residue connected to a solid support, the resulting protected dipeptide is also connected to the solid support. The protected dipeptide connected to the solid support may be advantageously used to add further amino acid residues to the N-terminus of the dipeptide by one or more deprotection and coupling cycles.

To enhance the rate of the coupling reaction in step b), it may be advantageous to use a carboxylic acid activating agent. In an embodiment, the present invention provides a method for preparing a dipeptide as defined herein, wherein step b) is performed in the presence of a carboxylic acid activating agent.

According to a fifth aspect, the present invention provides a method for preparing a protected oligopeptide or protected polypeptide, comprising preparing a protected dipeptide as defined in any one of the embodiments of the fourth aspect as disclosed herein, and comprising the further steps of:
c) deprotecting the C-terminus of the protected dipeptide, obtained in step b);
d) coupling the C-deprotected dipeptide obtained in step c) with a further alpha-amino acid compound, thereby obtaining the protected oligopeptide; and
e) optionally repeating steps c) and d) one or more times to add further amino acid residues to the protected oligopeptide obtained in step d).

In other words, the fifth aspect of the invention provides a method to prepare a protected oligopeptide or protected polypeptide by adding amino acid residues to the C-terminus of the protected dipeptide or oligopeptide. In this aspect, the N-terminus of the protected dipeptide or oligopeptide remains protected, while the C-terminus is deprotected and coupled with a further amino acid residue one or more times.

In a preferred embodiment of the fifth aspect, the present invention provides a method for preparing a protected oligopeptide or protected polypeptide, as defined herein, wherein the further amino acid residue is an alpha-amino acid ester or an alpha-amino acid bearing a carboxylic acid protecting group on its C-terminus.

According to a sixth aspect, the present invention provides a method for preparing a protected oligopeptide or protected polypeptide, comprising preparing a protected dipeptide as defined in any one of the embodiments of the fourth aspect as disclosed herein, and comprising the further steps of:
c) deprotecting the N-terminus of the protected dipeptide, obtained in step b), by contacting the protected dipeptide with a source of fluoride or hydroxide soluble in organic media, and subsequently with a nucleophile;
d) coupling the deprotected dipeptide obtained in step c) with a further alpha-amino acid compound, thereby obtaining the protected oligopeptide; and
e) optionally repeating steps c) and d) one or more times to add further amino acid residues to the protected oligopeptide obtained in step d).

It was found that the N-terminus of the protected dipeptide and/or the protected oligopeptide may be advantageously deprotected by contacting the protected dipeptide or oligopeptide with a source of fluoride or hydroxide soluble in organic media, thereby unblocking the protected amine moiety to provide the corresponding free isocyanate moiety in situ, and subsequently reacting or trapping the free isocyanate moiety with a nucleophile.

Any source of fluoride or hydroxide which is soluble in organic media may be used in the method according to the sixth aspect. It was found that tetraalkylammonium fluorides, such as tetrabutylammonium fluoride, and tetraalkylammonium hydroxides are particularly suitable, as they are soluble in most organic solvents or media.

Any nucleophile capable of reacting with the in situ formed free isocyanate may be used in the method according to the sixth aspect. It was however found that nucleophiles that result in a labile carbonyl moiety are particularly preferred, as they enable to provide the corresponding deprotected amine without any further steps. In a particular embodiment, the present invention provides a method for preparing a protected oligopeptide or protected polypeptide, as defined herein, wherein the nucleophile comprises water. When water is used, the in situ formed isocyanate is transformed into the corresponding carbamic acid, which on release of CO₂ provides the desired deprotected amine. It further appeared to be advantageous that the nucleophile is an acidic aqueous solution, in essence an aqueous solution with a pH below 7, as this may enhance the formation of the carbamic acid from the in situ formed free isocyanate.

The nucleophile may be present in the reaction mixture when the protected dipeptide is contacted with a source of fluoride or hydroxide soluble in organic media and/or the nucleophile may be added to the reaction mixture after the protected dipeptide is contacted with a source of fluoride or hydroxide soluble in organic media.

It was further found that the method according to the sixth aspect is particularly advantageous when the protected oligopeptide is connected to a solid support. In this embodiment, the solid support enables to improve the separation and purification of the protected oligopeptide during each of the deprotection and coupling cycles.

In the context of the present invention, the term 'solid support' refers to an insoluble polymer having free -OH or -NH₂ groups that are capable to react with an amino acid, or an activated amino acid. The free -OH or -NH₂ groups can be directly connected to the polymer backbone, or they can be connected to the polymer backbone via a linker.

A further advantage of the method according to the sixth aspect is that the pyrazole moiety is released during the step of contacting the protected dipeptide or protected oligopeptide with the source of fluoride or hydroxide soluble in organic media. With known protecting groups, like a fluorenylmethyloxycarbonyl (fmoc) protecting group, deprotection leads to the formation of a large amount of dibenzofulvene-piperidine adduct waste. In contrast, the pyrazole moiety released in the method according to the sixth aspect, may be recycled and used again in the method to prepare the protected amines, as described herein.

According to a seventh aspect, the present invention provides a method for preparing an oligopeptide or polypeptide, comprising preparing a protected oligopeptide or protected polypeptide as defined in any one of the embodiments of the fifth or sixth aspect as disclosed herein, and comprising the further steps of:
f) contacting the protected oligopeptide or protected polypeptide obtained in step d) or step e) with a source of fluoride or hydroxide soluble in organic media; and
g) contacting the compound obtained in step f) with a nucleophile.

Any nucleophile capable of reacting with the in situ formed free isocyanate may be used in the method according to the seventh aspect. It was however found that nucleophiles that result in a labile carbonyl moiety are particularly preferred, as they enable to provide the corresponding deprotected amine without any further steps, as mentioned hereinbefore.

Contacting the protected oligopeptide or protected polypeptide obtained in step d) or step e) with a source of fluoride or hydroxide soluble in organic media, thereby providing a compound comprising a free isocyanate moiety, and contacting said compound with a nucleophile, may take place in one or more steps. The nucleophile used in step g) may be present in the reaction mixture when the protected oligopeptide or protected polypeptide is contacted with a source of fluoride or hydroxide soluble in organic media, thereby in situ trapping the free isocyanate moiety of the obtained compound with the nucleophile. In other words, the activation or unblocking of the protected oligopeptide or protected polypeptide, and the reaction or trapping the free isocyanate moiety with the nucleophile, take place in the same step. Alternatively, or additionally, the nucleophile used in step g) may be added to the reaction mixture after the protected oligopeptide or protected polypeptide is contacted with a source of fluoride or hydroxide soluble in organic media. In other words, the activation or unblocking of the protected oligopeptide or protected polypeptide, and the reaction or trapping of the free isocyanate moiety with the nucleophile, take place in separate steps.

According to an eighth aspect, the present invention provides a protected oligopeptide or protected polypeptide represented by formula (V) 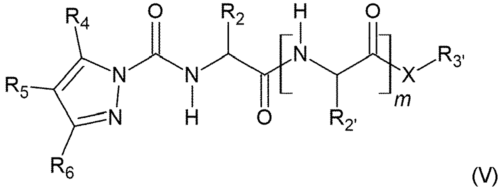 wherein
m is an integer from 1 to 100;
R₂ and each instance of R_{2'} are each independently selected from -H, -OH, -SH, SeH, - NR_{b}R_{b'}, -C₁₋₁₂alkyl, -C₂₋₁₂alkenyl, -C₃₋₇cycloalkyl, -CHO, -C(O)R_{c}, -COOH, -OC(O)R_{c}, - C(O)OR_{c}, -OC(O)OR_{c}, -NR_{b}C(O)R_{c}, -C(O)NR_{b}R_{b'}, -NR_{b}C(O)OR_{c}, and -OC(O)NR_{b}R_{b'}-, and Ar₂; wherein said -C₁₋₁₂alkyl, -C₂₋₁₂alkenyl, and -C₃₋₇cycloalkyl optionally comprises one or more heteroatoms selected from N, O, and S and/or is substituted with one or more substituents selected from -H, -OH, -SH, -NR_{d}R_{d'}, -C₁₋₁₂alkyl, -C₂₋₁₂alkenyl, -C₃₋₇cycloalkyl, - CHO, -C(O)Rₑ, -COOH, -OC(O)Rₑ, -C(O)ORₑ, -OC(O)ORₑ, -NR_{d}C(O)Rₑ, -C(O)NR_{d}R_{d'}, - NR_{d}C(O)ORₑ, -OC(O)NR_{d}R_{d'}-, and Ar₃;
each instance of R_{b}, each instance of R_{b'}, each instance of R_{c}, each instance of R_{d}, each instance of R_{d'} and each instance of Rₑ is independently selected from -H, and -C₁₋₁₂alkyl; Ar₂ and Ar₃ are each independently a 5- to 10-membered aromatic cycle optionally comprising one or more heteroatoms selected from O, N and S and/or substituted with one or more substituents independently selected from -halogen, -C₁₋₆alkyl, -C₂₋₆alkenyl, -C₃₋₇cycloalkyl, -OH, -SH, NR_{f}R_{f'}, and -OC₁₋₆alkyl;
each instance of R_{f} and each instance of R_{f'} is independently selected from -H, and -C₁₋₆alkyl;
X is selected from -O-, and -NH-;
R_{3'} is selected from -H, and -C₁₋₁₂alkyl, or R_{3'} is a solid support;
R₄, and R₆ are each independently selected from hydrogen, any halogen, any substituted or unsubstituted alkyl, any substituted or unsubstituted alkenyl, any substituted or unsubstituted cycloalkyl, and any substituted or unsubstituted aryl; and
R₅ is selected from hydrogen, hydroxy, thio, any substituted or unsubstituted amino, any substituted or unsubstituted alkoxy or aryloxy, any substituted or unsubstituted alkyl, any substituted or unsubstituted alkenyl, any substituted or unsubstituted cycloalkyl, any substituted or unsubstituted aryl, any substituted or unsubstituted ester, any substituted or unsubstituted carbonyl, any substituted or unsubstituted amide, any substituted or unsubstituted carbonate, and any substituted or unsubstituted carbamate.

The protected oligopeptide or protected polypeptide, as described herein, may contain any suitable number of alpha-amino acid residues, such as 2, 3, 4, 5, 6, 7, 8, 9, 10, 15, 20, 25, 30, 40, 50, 60, 70, 80, 90, 100, or more alpha-amino acid residues. In useful embodiments, the present invention provides the protected oligopeptide or protected polypeptide, as defined herein, wherein m is an integer from 2 to 100, preferably from 3 to 90, more preferably from 4 to 80, even more preferably from 5 to 70, yet even more preferably from 6 to 60, yet even more preferably from 7 to 50, yet even more preferably from 8 to 40, yet even more preferably m is an integer from 10 to 30.

In an embodiment, the present invention provides the protected oligopeptide or protected polypeptide as defined herein, and represented by formula (V) 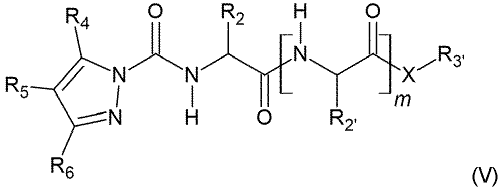 wherein
m is an integer from 1 to 100;
R₂ and each instance of R₂ are each independently selected from -H, -OH, -SH, SeH, - NR_{b}R_{b'}, -C₁₋₁₂alkyl, -C₂₋₁₂alkenyl, -C₃₋₇cycloalkyl, -CHO, -C(O)R_{c}, -COOH, -OC(O)R_{c}, - C(O)OR_{c}, -OC(O)OR_{c}, -NR_{b}C(O)R_{c}, -C(O)NR_{b}R_{b'}, -NR_{b}C(O)OR_{c}, and -OC(O)NR_{b}R_{b'}-, and Ar₂; wherein said -C₁₋₁₂alkyl, -C₂₋₁₂alkenyl, and -C₃₋₇cycloalkyl optionally comprises one or more heteroatoms selected from N, O, and S and/or is substituted with one or more substituents selected from -H, -OH, -SH, -NR_{d}R_{d'}, -C₁₋₁₂alkyl, -C₂₋₁₂alkenyl, -C₃₋₇cycloalkyl, - CHO, -C(O)Rₑ, -COOH, -OC(O)Rₑ, -C(O)ORₑ, -OC(O)ORₑ, -NR_{d}C(O)Rₑ, -C(O)NR_{d}R_{d'}, - NR_{d}C(O)ORₑ, -OC(O)NR_{d}R_{d'}-, and Ar₃;
X is selected from -O-, and -NH-;
R_{3'} is selected from -H, and -C₁₋₁₂alkyl, or R_{3'} is a solid support;
R₄ and R₆ are each independently selected from -H, -halogen, -C₁₋₁₂alkyl, -C₂₋₁₂alkenyl, -C₃₋₇cycloalkyl, and Ar₄; wherein said -C₁₋₁₂alkyl, -C₂₋₁₂alkenyl, and -C₃₋₇cycloalkyl optionally comprises one or more heteroatoms selected from N, O, and S and/or is substituted with one or more substituents selected from -halogen;
R₅ is selected from -H, -OH, -SH, -NR_{g}R_{g'}, -C₁₋₁₂alkyl, -C₂₋₁₂alkenyl, -C₃₋₇cycloalkyl, -CHO, - C(O)Rₕ, -COOH, -OC(O)Rₕ, -C(O)ORₕ, -OC(O)ORₕ, -NR_{g}C(O)Rₕ, -C(O)NR_{g}R_{g'}, - NR_{g}C(O)ORₕ, and -OC(O)NR_{g}R_{g'}-, and Ar₅; wherein said -C₁₋₁₂alkyl, -C₂₋₁₂alkenyl, and -C₃₋₇cycloalkyl optionally comprises one or more heteroatoms selected from N, O, and S and/or is substituted with one or more substituents selected from -H, -OH, -SH, -NRᵢR_{i'}, -C₁₋₁₂alkyl, -C₂₋₁₂alkenyl, -C₃₋₇cycloalkyl, -CHO, -C(O)Rⱼ, -COOH, -OC(O)Rⱼ, -C(O)ORⱼ, -OC(O)ORⱼ, - NRᵢC(O)Rⱼ, -C(O)NRᵢR_{i'}, -NRᵢC(O)ORⱼ, -OC(O)NRᵢR_{i'}-, and Ar₆;
Ar₂, Ar₃ Ar₄, Ar₅, and Ar₆ are each independently a 5- to 10-membered aromatic cycle optionally comprising one or more heteroatoms selected from O, N and S and/or substituted with one or more substituents independently selected from -halogen, -C₁₋₆alkyl, -C₂₋₆alkenyl, -C₃₋₇cycloalkyl, -OH, -SH, NR_{f}R_{f'}, and -OC₁₋₆alkyl;
each instance of R_{b}, each instance of R_{b'}, each instance of R_{c}, each instance of R_{d}, each instance of R_{d'}, each instance of Rₑ, each instance of R_{g}, each instance of R_{g'}, each instance of Rₕ, each instance of Rᵢ, each instance of R_{i'} and each instance of Rⱼ is independently selected from -H, and -C₁₋₁₂alkyl; and
each instance of R_{f} and each instance of R_{f'} is independently selected from -H, and -C₁₋₆alkyl.

### EXAMPLES

### Materials

Unless stated otherwise, all chemicals were purchased from commercial source, and used as received.

### Methods

¹H NMR spectra were recorded on a Bruker Avance III 400 (101 MHz for ¹³C) Fourier Transform NMR spectrometer at 300 K (unless stated otherwise), using the deuterated solvents CDCl₃ or DMSO-d₆ as internal standard (¹H: δ 7.26 ppm and ¹³C{¹H}: δ 77.16 ppm for CDCl₃; ¹H: δ 2.50 ppm and ¹³C{¹H}: δ 39.52 ppm for DMSO-d₆. Chemical shifts (δ) are reported in ppm; coupling constants (J) are reported in Hz; splitting patterns are assigned s = singlet, d = doublet, t = triplet, q = quartet, quint = quintet, sext = sextet, br = broad signal, m = multiplet or combinations thereof. ¹³C NMR spectra were recorded with complete proton decoupling. ¹H NMR yields were determined by addition of a known amount (on average 1/3 times the moles of the limiting reagent) of an internal standard (1 ,3,5-trimethoxybenzene, TMB) and dissolving everything in a suitable deuterated solvent, followed by ¹H NMR analysis.

### Synthesis of precursor C

### Example 1. Phenyl 3,5-dimethyl-1H-pyrazole-1-carboxylate

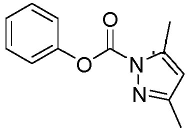

Diphenyl carbonate (42.8 g, 200 mmol), 3,5-dimethyl-1H-pyrazole (38.5 g, 400 mmol) and 4-dimethylaminopyridine (1.22 g, 10 mmol) were loaded to a reactor equipped with a magnetic stirring bar and a water-cooled cone-shaped collector. A vacuum pump is connected via a closed valve to the top of the reactor, in a manner that vapors pass through the cone before reaching the valve. The water cooling is turned on and the vacuum valve is closed before submerging the reactor in a water bath at 85 °C (internal temperature reactor = 80 °C). One hour after melting the solids under constant stirring, the vacuum valve was opened to lower the pressure in the reactor (p = 0.01 mbar). Solids start to deposit on the cone-shaped water cooling and the oily bottom fraction turns solid over time.

The solid residue in the bottom of the reactor is collected and analyzed confirming it is *title compound* (39.2 g, 91 %). The cone-shaped collector is rinsed with EtOAc (200 mL) after its full coverage with solids and the cleaning solution is transferred to a separatory funnel. The organic layer is washed once with 1 M HCl. The organic layer is dried over MgSO₄ and concentrated in vacuo to yield a fraction containing phenol contaminated with unreacted diphenyl carbonate (99 % recovery).

The aqueous layer was neutralized with a saturated aqueous solution of NaHCO₃ and was extracted with EtOAc (2 x 100 mL). The combined organic layers were dried over MgSO₄ and concentrated in vacuo to yield a fraction of 3,5-dimethyl-1H-pyrazole contaminated with 4-dimethylaminopyridine (93 % recovery based on the excess).

### Example 2. Phenyl 1H-imidazole-1-carboxylate

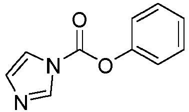

Diphenyl carbonate (214 mg, 1 mmol), 1H-imidazole (136 mg, 2 mmol) and DMAP (6 mg, 0.05 equiv.) were brought together in an oven dried pressure tube equipped with magnetic stirring bar and the contents were sealed with an aluminum crimp cap. The solids were heated to 80 °C and stirred at this temperature for 11 h. Analysis via ¹H NMR with TMB as internal standard indicated formation of the title compound (82 %) and phenol (87 %).

### Example 3. Phenyl 2-oxoazepane-1-carboxylate

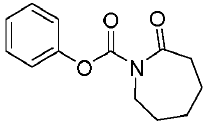

Diphenyl carbonate (42.8 g, 200 mmol), azepan-2-one (45.3 g, 400 mmol) and 4-Dimethylaminopyridine (1.222 g, 10.00 mmol) were loaded in a reactor equipped with a magnetic stirrer. The reactor was sealed, the solids were molten and the mixture was stirred at 100 °C for 16 h. The oily mixture was allowed to cool down to room temperature and was diluted with MTBE (300 mL). 5 subsequent washes with water (5 x 100 mL) were performed to remove the excess of azepan-2-one (34.1 g azepan-2-one was obtained after evaporation of the aqueous layer). The organic layer was washed twice more with 1 M NaOH (2 x 150 mL), dried over MgSO₄ and concentrated in vacuo to yield white solids. The solid residue was loaded in a sublimation apparatus and sublimed at 80 °C, p = 0.01 mbar. The solid residue in the bottom of the reactor is collected and analyzed confirming it is *title compound* (22 g, 47 %). From the cone-shaped condenser was collected diphenyl carbonate (18.7 g). The aqueous NaOH layer was acidified by addition of aqueous HCl (37 %) and was extracted twice by MTBE to yield phenol (11.1 g) after evaporation of the solvent.

### Example 4. Butan-2-one O-phenoxycarbonyl oxime

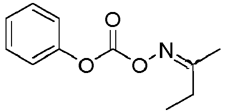

Diphenyl carbonate (171 g, 800 mmol) and 4-dimethylaminopyridine (1.22 g, 10 mmol) were added to a reactor heated at 80 °C to melt the solids. To the molten mixture, methyl ethyl ketone oxime (MEKO) (17.42 g, 200 mmol) was added over a short period of time. The resulting mixture was stirred at 80 °C for 1 h before cooling down the mixture to room temperature. To the solidified crude mixture was added 2.4 mL AcOH and 320 mL methanol washing the solids. Filtration yielded pure diphenyl carbonate as solids and the filtrate to which was added 80 mL water. After addition of the water, crystallization is observed. Filtration yielded pure diphenyl carbonate as solid and the filtrate was cooled to below 0 °C to induce more crystallization. Filtration yielded pure diphenyl carbonate as solid and the filtrate was diluted with 300 mL MTBE. The combined solid fraction were diphenyl carbonate (138.8 g). The organic layer was washed once with 1 M AcOH (150 mL), twice with 1 M NaOH (2 x 150 mL), dried over MgSO₄ and concentrated in vacuo to yield *title compound* as a transparent oil (27.2 g, 66 %). The aqueous NaOH layer was acidified by addition of aqueous HCl (37 %) and was extracted twice by MTBE to yield phenol (18.5 g) after evaporation of the solvent.

### Synthesis of protected amines from precursor C and a monoamine

### Example 5. Propan-2-one O-benzylcarbamoyl oxime

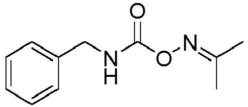

*O*,*O*'-carbonylbis(propan-2-one oxime) (172 mg, 1 mmol) was diluted with iPrOH (1 mL). To the solution was added benzylamine (161 mg, 1.5 mmol) and the resulting mixture was allowed to stir at 25 °C for 30 minutes. The mixture was diluted with MTBE and water.

The layers were separated and the organic layer was washed twice more with 1 M NaOH. Organics were dried over MgSO₄ and concentrated in vacuo to yield title compounds as a white solid (170 mg, 82 %).

### Example 6. Protected amines from phenyl 3,5-dimethyl-1H-pyrazole-1-carboxylate and a mono-amine

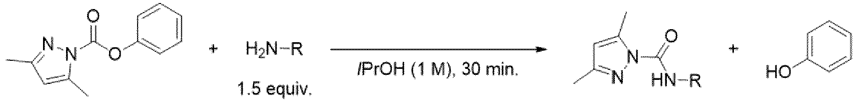

Phenyl 3,5-dimethyl-1H-pyrazole-1-carboxylate with a variety of mono-amines to obtain blocked isocyanates of formula here below (shown with respective yield):

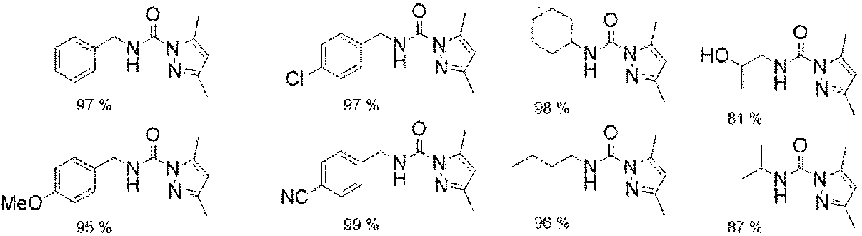

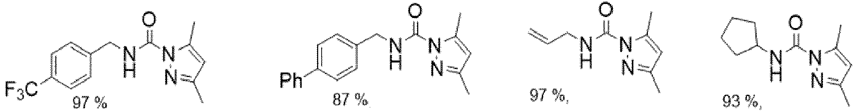

The blocked isocyanates above were obtained according to by reacting Phenyl 3,5-dimethyl-1H-pyrazole-1-carboxylate with the respective amine (1.5 eq.) in iPrOH (1 M) for 30 minutes.

### Example 7. N-benzyl-1H-imidazole-1-carboxamide

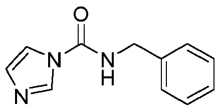

Phenyl 1*H*-imidazole-1-carboxylate (565 mg, 3 mmol) was brought together with *i*PrOH (3 mL) in a glass vial. To the stirred suspension was added benzylamine (327 µL, 3 mmol) and the mixture was stirred for 16 hr. MTBE and 1 M NaOH were added and the layers were separated before a second wash with 1 M NaOH. The organics were dried over MgSO₄ and concentrated in vacuo to yield *title compound* (501 mg, 83 %).

### Synthesis of protected amines from precursor C and primary diamines

### Example 8. N,N'-(hexane-1,6-diyl)bis(3,5-dimethyl-1H-pyrazole-1-carboxamide)

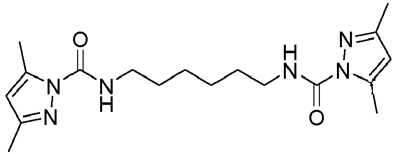

Hexamethylenediamine (1.45 g, 12.5 mmol) was diluted in *i*PrOH (25 mL) under magnetic stirring to yield a clear solution. To this solution was added phenyl 3,5-dimethyl-1*H*-pyrazole-1-carboxylate (5.41 g, 25 mmol) to form a suspension which dilutes overtime. After 16 h, the reaction was diluted with 1 M HCl and MTBE. The layers were separated and the organic layer was washed twice with 1 M NaOH. The organic layer was dried over MgSO₄ and concentrated in vacuo to yield *title compound* as a white solid (4.38 g, 97 %).The basic aqueous layer was acidified with 6 M HCl and was extracted with MTBE. The organic layer was dried over MgSO₄ and concentrated in vacuo to recover the phenol (99 %).

### Example 9. N-((5-(3,5-dimethyl-1H-pyrazole-1-carboxamido)-1,3,3-trimethylcyclohexyl)-methyl)-3,5-dimethyl-1H-pyrazole-1-carboxamide (cis- and trans-mixture)

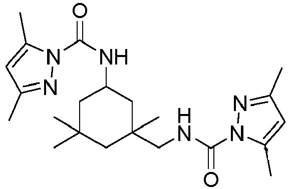

Isophoronediamine (2.129 g, 12.5 mmol) was diluted in iPrOH (25 mL) under magnetic stirring to yield a clear solution. To this solution was added phenyl 3,5-dimethyl-1*H*-pyrazole-1-carboxylate (5.41 g, 25 mmol) to form a suspension which dilutes overtime. After 16 h, the reaction was diluted with 1 M HCl and MTBE. The layers were separated and the organic layer was washed twice with 1 M NaOH. The organic layer was dried over MgSO₄ and concentrated in vacuo to yield *title compound* as a white solid (4.96 g, 96 %). The basic aqueous layer was acidified with 6 M HCl and was extracted with MTBE. The organic layer was dried over MgSO₄ and concentrated in vacuo to recover the phenol (94 %).

### Example 10. N,N-(methylenebis(cyclohexane-4,1-diyl))bis(3,5-dimethyl-1H-pyrazole-1- carboxamide) (cis- and trans-mixture)

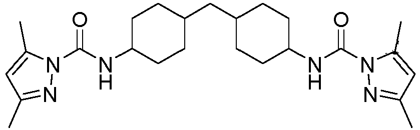

4,4'-methylenebis(cyclohexan-1-amine) (2.63 g, 12.5 mmol) was diluted in *i*PrOH (25 mL) under magnetic stirring to yield a clear solution. To this solution was added phenyl 3,5-dimethyl-1*H-*pyrazole-1-carboxylate (5.41 g, 25 mmol) to form a suspension which dilutes overtime. After 16 h, the reaction was diluted with 1 M HCl and MTBE. The layers were separated and the organic layer was washed twice with 1 M NaOH. The organic layer was dried over MgSO₄ and concentrated in vacuo to yield *title compound* as a white solid (5.39 g, 95 %).

The basic aqueous layer was acidified with 6 M HCl and was extracted with MTBE. The organic layer was dried over MgSO₄ and concentrated in vacuo to recover the phenol (99 %).

### Example 11. N,N'-(pentane-1,5-diyl)bis(3,5-dimethyl-1H-pyrazole-1-carboxamide)

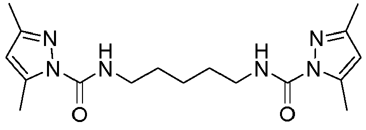

Pentamethylenediamine (1.28 g, 12.5 mmol) was diluted in *i*PrOH (25 mL) under magnetic stirring to yield a clear solution. To this solution was added phenyl 3,5-dimethyl-1*H*-pyrazole-1-carboxylate (5.41 g, 25 mmol) to form a suspension which dilutes over time. After 16 h, the reaction was diluted with 1 M HCl and MTBE. The layers were separated and the organic layer was washed twice with 1 M NaOH. The organic layer was dried over MgSO₄ and concentrated in vacuo to yield *title compound* as a white solid (4.33 g, 100 %).

The basic aqueous layer was acidified with 6 M HCl and was extracted with MTBE. The organic layer was dried over MgSO₄ and concentrated in vacuo to recover the phenol (89 %).

### Example 12. N,N'-(ethane-1,2-diyl)bis(3,5-dimethyl-1H-pyrazole-1-carboxamide)

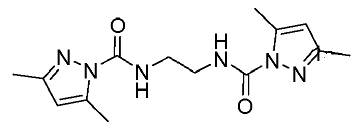

Ethane-1,2-diamine (0.75 g, 12.5 mmol) was diluted in *i*PrOH (25 mL) under magnetic stirring to yield a clear solution. To this solution was added phenyl 3,5-dimethyl-1*H*-pyrazole-1-carboxylate (5.41 g, 25 mmol) to form a suspension which dilutes over time. After 16 h, the reaction was diluted with 1 M HCl and MTBE. The layers were separated and the organic layer was washed twice with 1 M NaOH. The organic layer was dried over MgSO₄ and concentrated in vacuo to yield *title compound* as a white solid (3.85 g, 100 %).

The basic aqueous layer was acidified with 6 M HCl and was extracted with MTBE. The organic layer was dried over MgSO₄ and concentrated in vacuo to recover the phenol (100 %).

### Example 13. N,N'-(hexane-1,6-diyl)bis(2-oxoazepane-1-carboxamide)

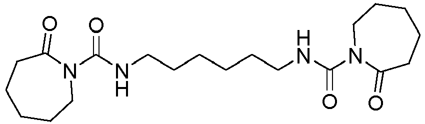

Hexamethylenediamine (1.45 g, 12.5 mmol) was diluted in *i*PrOH (25 mL) under magnetic stirring to yield a clear solution. To this solution was added phenyl 2-oxoazepane-1-carboxylate (5.83 g, 25 mmol) to form a suspension which dilutes over time. After 16 h, the reaction was diluted with 1 M HCl and MTBE. The layers were separated and the organic layer was washed twice with 1 M NaOH. The organic layer was dried over MgSO₄ and concentrated in vacuo to yield *title compound* as a white solid (4.66 g, 94 %).

The basic aqueous layer was acidified with 6 M HCl and was extracted with MTBE. The organic layer was dried over MgSO₄ and concentrated in vacuo to recover the phenol (78 %).

### Example 14. 2-oxo-N-(3,3,5-trimethyl-5-((2-oxoazepane-1-carboxamido)methyl)cyclohexyl)azepane-1-carboxamide (cis- and trans-mixture)

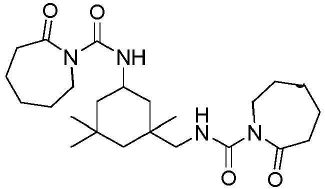

Isophoronediamine (2.129 g, 12.5 mmol) was diluted in *i*PrOH (25 mL) under magnetic stirring to yield a clear solution. To this solution was added phenyl 2-oxoazepane-1-carboxylate (5.83 g, 25 mmol) to form a suspension which dilutes overtime. After 16 h, the reaction was diluted with 1 M HCl and MTBE. The layers were separated and the organic layer was washed twice with 1 M NaOH. The organic layer was dried over MgSO₄ and concentrated in vacuo to yield *title compound* as a white solid (5.5 g, 98 %).

The basic aqueous layer was acidified with 6 M HCl and was extracted with MTBE. The organic layer was dried over MgSO₄ and concentrated in vacuo to recover the phenol (77 %).

### Example 15. O,O'-((hexane-1,6-diylbis(azanediyl))bis(carbonyl))bis(butan-2-one oxime)

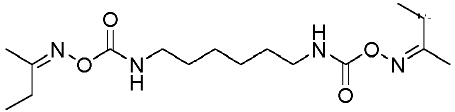

Hexamethylenediamine (1.45 g, 12.5 mmol) was diluted in *i*PrOH (25 mL) under magnetic stirring to yield a clear solution. To this solution was added Butan-2-one *O-*phenoxycarbonyl oxime (5.18 g, 25 mmol) to form a clear yellow solution. After 16 h, the reaction was diluted with 1 M AcOH and MTBE. The layers were separated and the organic layer was washed twice with 1 M NaOH. The organic layer was dried over MgSO₄ and concentrated in vacuo to yield *title compound* as a yellowish solid (3.9 g, 91 %).

The basic aqueous layer was acidified with 6 M HCl and was extracted with MTBE. The organic layer was dried over MgSO₄ and concentrated in vacuo to recover the phenol (98 %).

### Example 16. Butan-2-one O-(((5-(((((-butan-2-ylidene)amino)oxy)carbonyl)amino)-1,3,3-trimethylcyclohexyl)methyl)carbamoyl) oxime (cis- and trans-mixture)

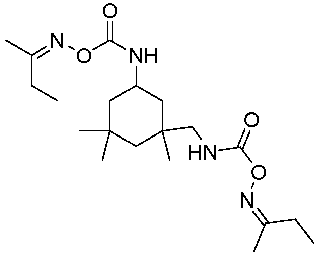

Isophoronediamine (2.129 g, 12.5 mmol) was diluted in *i*PrOH (25 mL) under magnetic stirring to yield a clear solution. To this solution was added Butan-2-one *O-*phenoxycarbonyl oxime (5.18 g, 25 mmol) to form a clear yellow solution. After 16 h, the reaction was diluted with 1 M AcOH and MTBE. The layers were separated and the organic layer was washed twice with 1 M NaOH. The organic layer was dried over MgSO₄ and concentrated in vacuo to yield *title compound* as a yellowish solid (4.7 g, 95 %).

The basic aqueous layer was acidified with 6 M HCl and was extracted with MTBE. The organic layer was dried over MgSO₄ and concentrated in vacuo to recover the phenol (80 %).

### Example 17. O,O'-(((methylenebis(cyclohexane-4,1-diyl))bis(azanediyl))bis(carbonyl))-bis(butan-2-one oxime) (cis- and trans-mixture)

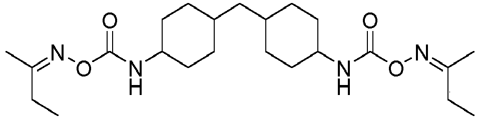

4,4'-methylenebis(cyclohexan-1-amine) (2.63 g, 12.5 mmol) was diluted in *i*PrOH (25 mL) under magnetic stirring to yield a clear solution. To this solution was added Butan-2-one *O*-phenoxycarbonyl oxime (5.18 g, 25 mmol) to form a clear yellow solution. After 16 hours, the reaction was diluted with 1 M AcOH and MTBE. The layers were separated and the organic layer was washed twice with 1 M NaOH. The organic layer was dried over MgSO₄ and concentrated in vacuo to yield *title compound* as a yellowish solid (5.38 g, 99 %).

The basic aqueous layer was acidified with 6 M HCl and was extracted with MTBE. The organic layer was dried over MgSO₄ and concentrated in vacuo to recover the phenol (87 %).

### Example 18. N,N'-(methylenebis(4,1-phenylene))bis(3,5-dimethyl-1H-pyrazole-1-carboxamide)

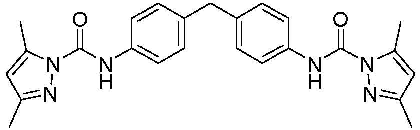

DBU (3.77 mL, 25.00 mmol) was dissolved in EtOAc (50 mL) to which was added 4,4'-Diaminodiphenylmethane (2.478 g, 12.50 mmol) and phenyl 3,5-dimethyl-1*H*-pyrazole-1-carboxylate (6.49 g, 30 mmol). The resulting solution was mixed at 5 °C for 24 h. The suspension was filtrated at 5 °C and the solids were washed thoroughly with water before filtration. The solids were washed once more with low amounts of methanol and acetone before washing again with water. The solids were dried at high vacuum and proved to be *title compound* (5.36 g, 97 %).

The filtrate was diluted with EtOAc and 1 M HCl. The layers were separated and the organic layer was dried over MgSO₄ and concentrated in vacuo to yield phenol (quant.).

### Example 19. N,N'-(methylenebis(4,1 -phenylene))bis(2-oxoazepane-1 -carboxamide)

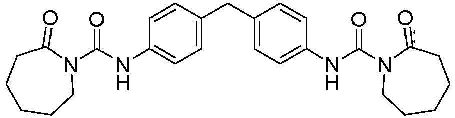

DBU (3.77 mL, 25.00 mmol) was dissolved in EtOAc (50 mL) to which was added 4,4'-Diaminodiphenylmethane (2.478 g, 12.50 mmol) and phenyl 2-oxoazepane-1-carboxylate (7.00 g, 30 mmol). The resulting solution was mixed at 25 °C for 24 h. The solvent was removed from the suspension *in vacuo* and the solids were washed thoroughly with water before filtration. The solids were washed once more with low amounts of methanol and acetone before washing again with water. The solids were dried at high vacuum and proved to be *title compound* (3.8 g, 72 %).

The filtrate was diluted with EtOAc and 1 M HCl. The layers were separated and the organic layer was dried over MgSO₄ and concentrated in vacuo to yield phenol (77 %).

### Example 20. O,O'-(((methylenebis(4,1-phenylene))bis(azanediyl))bis(carbonyl))bis(butan-2-one oxime)

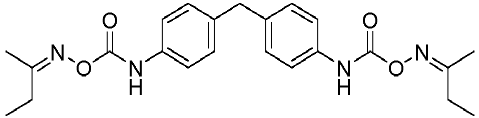

DBU (3.77 mL, 25.00 mmol) was dissolved in EtOAc (50 mL) to which was added 4,4'-Diaminodiphenylmethane (2.478 g, 12.50 mmol) and butan-2-one *O-*phenoxycarbonyl oxime (6.22 g, 30 mmol). The resulting solution was mixed at 25 °C for 24 h. The solvent was removed from the suspension *in vacuo* and the solids were washed thoroughly with water before filtration. The solids were washed once more with low amounts of methanol and acetone before washing again with water. The solids were dried at high vacuum and proved to be *title compound* (5.31 g, 89 %).

The filtrate was diluted with EtOAc and 1 M HCl. The layers were separated and the organic layer was dried over MgSO₄ and concentrated in vacuo to yield phenol (quant.).

### Example 21. N,N'-(hexane-1,6-diyl)bis(3,5-dimethyl-1H-pyrazole-1-carboxamide)

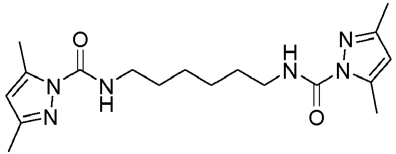

0.105 mL of a 60 wt% solution of hexamethylenediamine (0.5 mmol) in water was added to a suspension of phenyl 3,5-dimethyl-1H-pyrazole-1-carboxylate (216 mg, 1 mmol) in water (3 mL) with a few added droplets of THF. The reaction was left running at room temperature. After 16 h, the reaction was diluted with 1 M HCl and MTBE. The layers were separated and the organic layer was washed twice with 1 M NaOH. The organic layer was dried over MgSO₄ and concentrated in vacuo to yield *title compound* as a white solid (170 mg, 94 %).The basic aqueous layer was acidified with 6 M HCl and was extracted with MTBE. The organic layer was dried over MgSO₄ and concentrated in vacuo to recover the phenol (quant.).

### Example 22. N,N'-(hexane-1,6-diyl)bis(2-oxoazepane-1-carboxamide)

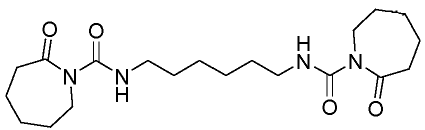

0.105 mL of a 60 wt% solution of hexamethylenediamine (0.5 mmol) in water was added to a suspension of 2-oxoazepane-1-carboxylate (233 mg, 1 mmol) in water (3 mL) with a few added droplets of THF. The reaction was left running at room temperature. After 16 h, the reaction was diluted with 1 M HCl and MTBE. The layers were separated and the organic layer was washed twice with 1 M NaOH. The organic layer was dried over MgSO₄ and concentrated in vacuo to yield *title compound* as a white solid (203 mg, 98 %).The basic aqueous layer was acidified with 6 M HCl and was extracted with MTBE. The organic layer was dried over MgSO₄ and concentrated in vacuo to recover the phenol (97 %).

### Example 23. O,O'-((hexane-1,6-diylbis(azanediyl))bis(carbonyl))bis(butan-2-one oxime)

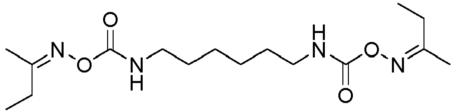

0.105 mL of a 60 wt% solution of hexamethylenediamine (0.5 mmol) in water was added to an emulsion of butan-2-one *O-*phenoxycarbonyl oxime (207 mg, 1 mmol) in water (3 mL) with a few added droplets of THF. The reaction was left running at room temperature. After 16 h, the reaction was diluted with 1 M HCl and MTBE. The layers were separated and the organic layer was washed twice with 1 M NaOH. The organic layer was dried over MgSO₄ and concentrated in vacuo to yield *title compound* as a white solid (139 mg, 81 %).The basic aqueous layer was acidified with 6 M HCl and was extracted with MTBE. The organic layer was dried over MgSO₄ and concentrated in vacuo to recover the phenol (65 %).

### Synthesis of protected amines from precursor C and an amino acid

### Example 24. (3,5-Dimethyl-1H-pyrazole-1-carbonyl)glycine

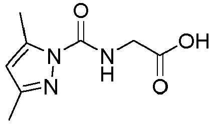

Phenyl 3,5-dimethyl-1*H*-pyrazole-1-carboxylate (324 mg, 1.500 mmol) and glycine (75 mg, 1 mmol) were dissolved in THF/1 M NaOH (1/1, 3 mL) and allowed to stir for 16 h at 25 °C. The mixture was diluted with MTBE and 1 M HCl. The layers were separated and the organic layer was washed with sat aq. NaHCOs. The layers were separated and the NaHCOs was acidified again to pH = 2 and extracted with MTBE. The last organic layer was dried over MgSO4 and concentrated in vacuo to yield *title compound* as a white solid (144 mg, 73 %) which solidified over a long period of time.

### Example 25. (3,5-dimethyl-1H-pyrazole-1-carbonyl)phenylalanine

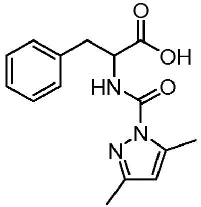

L-Phenylalanine (165 mg, 1 mmol) was dissolved in tetrabutylammonium hydroxide (as a solution of 40 %wt in water, 0.65 mL, 1 mmol) and THF (2 mL) was added to obtain a clear transparent solution. Then phenyl 3,5-dimethyl-1H-pyrazole-1-carboxylate (324 mg, 1.5 mmol) was added and the resulting suspension was allowed to stir at room temperature for 2 h. 1 M HCl (aq.) and EtOAc were added to quench the reaction and the layers were separated. The organic layer was dried over MgSO₄ and concentrated in vacuo. Analysis via ¹H NMR with TMB internal standard indicated formation of the title compound in 91 % yield.

### Example 26. (3,5-dimethyl-1H-pyrazole-1-carbonyl)serine

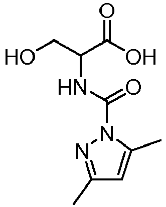

Serine (105 mg, 1 mmol) was dissolved in tetrabutylammonium hydroxide (as a solution of 40 %wt in water, 0.65 mL, 1 mmol) and THF (2 mL) was added to obtain a clear transparent solution. Then phenyl 3,5-dimethyl-1*H-*pyrazole-1-carboxylate (324 mg, 1.5 mmol) was added and the resulting suspension was allowed to stir at room temperature for 2 h. 1 M HCl (aq.) and EtOAc were added to quench the reaction and the layers were separated. The organic layer was dried over MgSO₄ and concentrated in vacuo. Analysis via ¹H NMR with TMB internal standard indicated formation of the title compound in 86 % yield.

### Example 27. (3,5-dimethyl-1H-pyrazole-1-carbonyl)cysteine

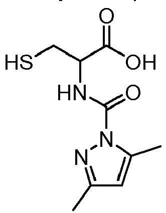

Cysteine (121 mg, 1 mmol) was dissolved in tetrabutylammonium hydroxide (as a solution of 40 %wt in water, 0.65 mL, 1 mmol) and THF (2 mL) was added to obtain a clear transparent solution. Then phenyl 3,5-dimethyl-1*H-*pyrazole-1-carboxylate (227 mg, 1.05 mmol) was added and the resulting suspension was allowed to stir at room temperature for 2 h. 1 M HCl (aq.) and EtOAc were added to quench the reaction and the layers were separated. The organic layer was dried over MgSO₄ and concentrated in vacuo. Analysis via ¹H NMR with TMB internal standard indicated formation of the title compound in 74 % yield.

### Example 28. (3,5-dimethyl-1H-pyrazole-1-carbonyl)-glutamic acid

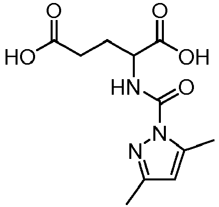

Glutamic acid (147 mg, 1 mmol) was dissolved in tetrabutylammonium hydroxide (as a solution of 40 %wt in water, 1.30 mL, 2 mmol) and acetone (2 mL) was added to obtain a clear transparent solution. Then phenyl 3,5-dimethyl-1*H*-pyrazole-1-carboxylate (324 mg, 1.5 mmol) was added and the resulting suspension was allowed to stir at room temperature for 4 h. 1 M HCl (aq.) and EtOAc were added to quench the reaction and the layers were separated. The organic layer was dried over MgSO₄ and concentrated in vacuo. Analysis via ¹H NMR with TMB internal standard indicated formation of the title compound in 91 % yield.

### Example 29. (3,5-dimethyl-1H-pyrazole-1-carbonyl)-aspartic acid

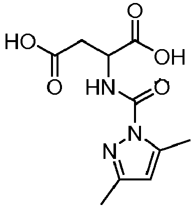

Aspartic acid (133 mg, 1 mmol) was dissolved in tetrabutylammonium hydroxide (as a solution of 40 %wt in water, 1.30 mL, 2 mmol) and acetone (2 mL) was added to obtain a clear transparent solution. Then phenyl 3,5-dimethyl-1*H*-pyrazole-1-carboxylate (324 mg, 1.5 mmol) was added and the resulting suspension was allowed to stir at room temperature for 4 h. 1 M HCl (aq.) and EtOAc were added to quench the reaction and the layers were separated.

The organic layer was dried over MgSO₄ and concentrated in vacuo. Analysis via ¹H NMR with TMB internal standard indicated formation of the title compound in 99 % yield.

### Example 30. (3,5-dimethyl-1H-pyrazole-1-carbonyl)-glutamine

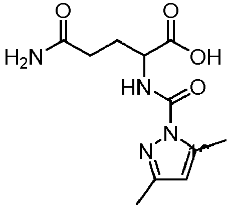

Glutamine (146 mg, 1 mmol) was dissolved in tetrabutylammonium hydroxide (as a solution of 20 %wt in water, 1.30 mL, 1 mmol) and acetone (2 mL) was added to obtain a clear transparent solution. Then phenyl 3,5-dimethyl-1*H*-pyrazole-1-carboxylate (324 mg, 1.5 mmol) was added and the resulting suspension was allowed to stir at room temperature for 4 h. The reaction mixture was concentrated in vacuo at room temperature. Analysis via ¹H NMR with TMB internal standard indicated formation of the title compound in 90 % yield.

### Example 31. (3,5-dimethyl-1H-pyrazole-1-carbonyl)-asparagine

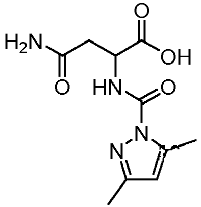

Asparagine (132 mg, 1 mmol) was dissolved in tetrabutylammonium hydroxide (as a solution of 20 %wt in water, 1.30 mL, 1 mmol) and acetone (2 mL) and DMSO (2 mL) were added to obtain a clear transparent solution. Then phenyl 3,5-dimethyl-1*H*-pyrazole-1-carboxylate (324 mg, 1.5 mmol) was added and the resulting suspension was allowed to stir at room temperature for 4 h. The reaction mixture was concentrated in vacuo at room temperature. Analysis via ¹H NMR with TMB internal standard indicated formation of the title compound in 77 % yield.

### Example 32. N2,N6-bis(3,5-dimethyl-1H-pyrazole-1-carbonyl) lysine

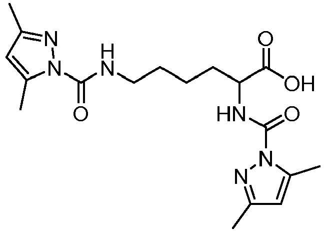

Lysine (146 mg, 1 mmol) was dissolved in tetrabutylammonium hydroxide (as a solution of 40 %wt in water, 0.65 mL, 1 mmol) and acetone (2 mL) was added to obtain a clear transparent solution. Then phenyl 3,5-dimethyl-1*H*-pyrazole-1-carboxylate (454 mg, 2.1 mmol) was added and the resulting suspension was allowed to stir at room temperature for 4 h. The reaction mixture was concentrated in vacuo at room temperature. Analysis via ¹H NMR with TMB internal standard indicated formation of the title compound in 88 % yield.

### Example 33. (1H-pyrazole-1-carbonyl) phenylalanine

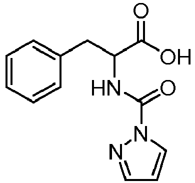

Phenylalanine (82 mg, 0.50 mmol) was dissolved in tetrabutylammonium hydroxide (as a solution of 40 %wt in water, 0.322 mL, 0.50 mmol) and acetone (1 mL). Then phenyl 1H-pyrazole-1-carboxylate (93 mg, 0.5 mmol) was added and the resulting suspension was allowed to stir at room temperature for 3 h. 1 M HCl (aq.) and EtOAc were added to quench the reaction and the layers were separated. The organic layer was dried over MgSO₄ and concentrated in vacuo. Analysis via ¹H NMR with TMB internal standard indicated formation of the title compound in 57 % yield.

### Example 34. (5-phenyl-1H-pyrazole-1-carbonyl) phenylalanine and (3-phenyl-1H-pyrazole-1-carbonyl)phenylalanine (mixture of isomers)

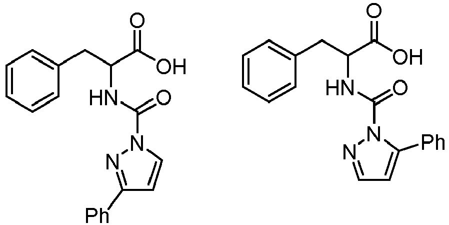

Phenylalanine (40 mg, 0.24 mmol) was dissolved in tetrabutylammonium hydroxide (as a solution of 40 %wt in water, 0.157 mL, 0.24 mmol) and acetone (0.5 mL). Then phenyl 5-phenyl-1H-pyrazole-1-carboxylate (64 mg, 0.24 mmol) was added and the resulting suspension was allowed to stir at room temperature for 3 h. 1 M HCl (aq.) and EtOAc were added to quench the reaction and the layers were separated. The organic layer was dried over MgSO₄ and concentrated in vacuo. Analysis via ¹H NMR with TMB internal standard indicated formation of the title compound in 73 % yield.

### Example 35. (4-bromo-3,5-dimethyl-1H-pyrazole-1-carbonyl)phenylalanine

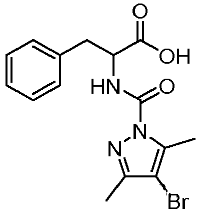

Phenylalanine (96 mg, 0.58 mmol) was dissolved in tetrabutylammonium hydroxide (as a solution of 40 %wt in water, 0.377 mL, 0.58 mmol) and acetone (1.2 mL). Then phenyl 4-bromo-3,5-dimethyl-1H-pyrazole-1-carboxylate (172 mg, 0.58 mmol) was added and the resulting suspension was allowed to stir at room temperature for 3 h. 1 M HCl (aq.) and EtOAc were added to quench the reaction and the layers were separated. The organic layer was dried over MgSO₄ and concentrated in vacuo. Analysis via ¹H NMR with TMB internal standard indicated formation of the title compound in 59 % yield.

### Example 36. (3-(trifluoromethyl)-1H-pyrazole-1-carbonyl)phenylalanine and (5-(trifluoromethyl)-1H-pyrazole-1-carbonyl)phenylalanine (mixture of isomers)

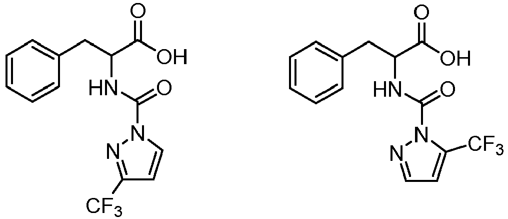

Phenylalanine (74 mg, 0.45 mmol) was dissolved in tetrabutylammonium hydroxide (as a solution of 40 %wt in water, 0.291 mL, 0.45 mmol) and acetone (0.9 mL). Then phenyl 3-(trifluoromethyl)-1H-pyrazole-1-carboxylate (115 mg, 0.45 mmol) was added and the resulting suspension was allowed to stir at room temperature for 3 h. 1 M HCl (aq.) and EtOAc were added to quench the reaction and the layers were separated. The organic layer was dried over MgSO₄ and concentrated in vacuo. Analysis via ¹H NMR with TMB internal standard indicated formation of the title compound in 56 % yield.

### Synthesis of protected oligopeptides from a protected amine and a further amino acid compound

### Example 37. Methyl (3,5-dimethyl-1H-pyrazole-1-carbonyl)-L-phenylalanyl-L-alaninate

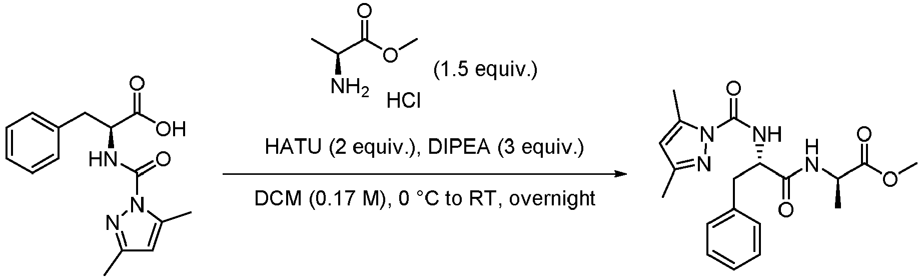

(3,5-dimethyl-1H-pyrazole-1-carbonyl)-L-phenylalanine (86 mg, 0.3 mmol) and HATU (228 mg, 0.600 mmol) were brought in a vial and cooled with an ice-bath. Then DIPEA (0.157 ml, 0.900 mmol) and DCM (1.8 mL) were added and allowed to react for 10 minutes. Then L-alanine methyl ester hydrochloride (63 mg, 0.450 mmol) was added and the reaction was allowed to warm up to room temperature for overnight reaction. Dilution with EtOAc and transferred to a separatory funnel. The organic layer was washed thrice with 1M HCl, once washed with 1M Na₂CO₃ followed by a final wash with brine. Organics were dried over MgSO₄, concentrated in vacuo. Analysis via ¹H NMR with TMB internal standard indicated formation of the *title compound* in 84 % yield.

### REFERENCES

1. Suppo, J-S; Subra, G.; Bergès, M; Marcia de Figueiredo, R.; Campagne, J-M Angew. Chem. Int. Ed. 2014, 53, 5389-5393

## Claims

1. A method for preparing a protected amine, the method comprising the steps of:
a) providing an organic carbonate A represented by formula (I) 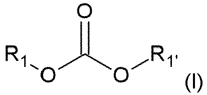 wherein
R₁ and R_{1'} are each independently selected from any substituted or unsubstituted alkyl, any substituted or unsubstituted alkenyl, any substituted or unsubstituted cycloalkyl, and any substituted or unsubstituted aryl; or
R₁ and R_{1'}, together with the -O-C-O- fragment to which they are attached, form one or more cyclic structures;
with the proviso that R₁ and R_{1'} are not -CCl₃;
b) reacting, in a liquid medium, the organic carbonate A provided in step a) with an active hydrogen compound B, thereby obtaining a precursor C; wherein the active hydrogen compound B is selected from substituted or unsubstituted azoles, substituted or unsubstituted secondary amides, substituted or unsubstituted lactams, and substituted or unsubstituted oximes, preferably substituted or unsubstituted azoles, more preferably substituted or unsubstituted pyrazole; and
c) reacting the precursor C obtained in step b) with an amine D having one or more -NH₂ groups, thereby obtaining the protected amine.

2. The method as claimed in claim 1, wherein the organic carbonate A is selected from diphenyl carbonate, dimethyl carbonate, diethyl carbonate, ethylene carbonate, propylene carbonate and trimethylene carbonate; in particular wherein the organic carbonate A is diphenyl carbonate.

3. The method as claimed in claim 1 or 2, wherein the active hydrogen compound B is selected from ε-caprolactam, 3,5-dimethylpyrazole, acetoxime, and 2-butanone oxime.

4. The method as claimed in claim 1 or 2, wherein the active hydrogen compound B is represented by formula (II) 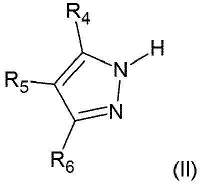 wherein
R₄, and R₆ are each independently selected from hydrogen, any halogen, any substituted or unsubstituted alkyl, any substituted or unsubstituted alkenyl, any substituted or unsubstituted cycloalkyl, and any substituted or unsubstituted aryl; and
R₅ is selected from hydrogen, hydroxy, thio, any substituted or unsubstituted amino, any substituted or unsubstituted alkoxy or aryloxy, any substituted or unsubstituted alkyl, any substituted or unsubstituted alkenyl, any substituted or unsubstituted cycloalkyl, any substituted or unsubstituted aryl, any substituted or unsubstituted ester, any substituted or unsubstituted carbonyl, any substituted or unsubstituted amide, any substituted or unsubstituted carbonate, and any substituted or unsubstituted carbamate.

5. The method as claimed in any one of claims 1 to 4, wherein the amine D is selected from any monoamine, any diamine, any triamine, and any polyamine; preferably the amine D is an amino acid, an amino acid ester, or an alkanol amine; more preferably the amine D is an alpha-amino acid or an alpha-amino acid ester.

6. The method as claimed in any one of claims 1 to 5, wherein the amine D is an alpha-amino acid or an alpha-amino acid ester represented by formula (III) 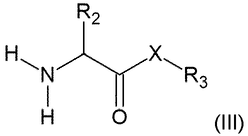 wherein
R₂ is selected from -H, -OH, -SH, SeH, -NR_{b}R_{b'}, -C₁₋₁₂alkyl, -C₂₋₁₂alkenyl, -C₃₋₇cycloalkyl, - CHO, -C(O)R_{c}, -COOH, -OC(O)R_{c}, -C(O)OR_{c}, -OC(O)OR_{c}, -NR_{b}C(O)R_{c}, -C(O)NR_{b}R_{b'}, - NR_{b}C(O)OR_{c}, and -OC(O)NR_{b}R_{b'}-, and Ar₂; wherein said -C₁₋₁₂alkyl, -C₂₋₁₂alkenyl, and -C₃₋₇cycloalkyl optionally comprises one or more heteroatoms selected from N, O, and S and/or is substituted with one or more substituents selected from -H, -OH, -SH, -NR_{d}R_{d'}, -C₁₋₁₂alkyl, -C₂₋₁₂alkenyl, -C₃₋₇cycloalkyl, -CHO, -C(O)Rₑ, -COOH, -OC(O)Rₑ, -C(O)ORₑ, -OC(O)ORₑ, - NR_{d}C(O)Rₑ, -C(O)NR_{d}R_{d'}, -NR_{d}C(O)ORₑ, -OC(O)NR_{d}R_{d'}-, and Ar₃;
each instance of R_{b}, each instance of R_{b'}, each instance of R_{c}, each instance of R_{d}, each instance of R_{d'} and each instance of Rₑ is independently selected from -H, and -C₁₋₁₂alkyl; Ar₂ and Ar₃ are each independently a 5- to 10-membered aromatic cycle optionally comprising one or more heteroatoms selected from O, N and S and/or substituted with one or more substituents independently selected from -halogen, -C₁₋₆alkyl, -C₂₋₆alkenyl, -C₃₋₇cycloalkyl, -OH, -SH, NR_{f}R_{f'}, and -OC₁₋₆alkyl;
each instance of R_{f} and each instance of R_{f'} is independently selected from -H, and -C₁₋₆alkyl;
X is selected from -O-, and -NH-; and
R₃ is selected from -H, and -C₁₋₁₂alkyl, or R₃ is a solid support.

7. The method as claimed in any one of claims 1 to 6, wherein step b) is performed in the presence of a nucleophilic catalyst.

8. The method as claimed in any one of claims 1 to 7, wherein in step b) the liquid medium is the organic carbonate A or a solvent.

9. A protected amine, in particular a protected amino acid or amino acid ester, represented by formula (IV) 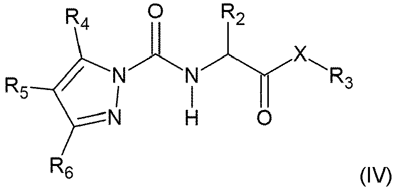 wherein
R₂ is selected from -H, -OH, -SH, SeH, -NR_{b}R_{b'}, -C₁₋₁₂alkyl, -C₂₋₁₂alkenyl, -C₃₋₇cycloalkyl, - CHO, -C(O)R_{c}, -COOH, -OC(O)R_{c}, -C(O)OR_{c}, -OC(O)OR_{c}, -NR_{b}C(O)R_{c}, -C(O)NR_{b}R_{b'}, - NR_{b}C(O)OR_{c}, and -OC(O)NR_{b}R_{b'}-, and Ar₂; wherein said -C₁₋₁₂alkyl, -C₂₋₁₂alkenyl, and -C₃₋₇cycloalkyl optionally comprises one or more heteroatoms selected from N, O, and S and/or is substituted with one or more substituents selected from -H, -OH, -SH, -NR_{d}R_{d'}, -C₁₋₁₂alkyl, -C₂₋₁₂alkenyl, -C₃₋₇cycloalkyl, -CHO, -C(O)Rₑ, -COOH, -OC(O)Rₑ, -C(O)ORₑ, -OC(O)ORₑ, - NR_{d}C(O)Rₑ, -C(O)NR_{d}R_{d'}, -NR_{d}C(O)ORₑ, -OC(O)NR_{d}R_{d'}-, and Ar₃;
each instance of R_{b}, each instance of R_{b'}, each instance of R_{c}, each instance of R_{d}, each instance of R_{d'} and each instance of Rₑ is independently selected from -H, and -C₁₋₁₂alkyl; Ar₂ and Ar₃ are each independently a 5- to 10-membered aromatic cycle optionally comprising one or more heteroatoms selected from O, N and S and/or substituted with one or more substituents independently selected from -halogen, -C₁₋₆alkyl, -C₂₋₆alkenyl, -C₃₋₇cycloalkyl, -OH, -SH, NR_{f}R_{f'}, and -OC₁₋₆alkyl;
each instance of R_{f} and each instance of R_{f'} is independently selected from -H, and -C₁₋₆alkyl;
X is selected from -O-, and -NH-;
R₃ is selected from -H, and -C₁₋₁₂alkyl, or R₃ is a solid support;
R₄, and R₆ are each independently selected from hydrogen, any halogen, any substituted or unsubstituted alkyl, any substituted or unsubstituted alkenyl, any substituted or unsubstituted cycloalkyl, and any substituted or unsubstituted aryl; and
R₅ is selected from hydrogen, hydroxy, thio, any substituted or unsubstituted amino, any substituted or unsubstituted alkoxy or aryloxy, any substituted or unsubstituted alkyl, any substituted or unsubstituted alkenyl, any substituted or unsubstituted cycloalkyl, any substituted or unsubstituted aryl, any substituted or unsubstituted ester, any substituted or unsubstituted carbonyl, any substituted or unsubstituted amide, any substituted or unsubstituted carbonate, and any substituted or unsubstituted carbamate.

10. A protected amine, as claimed in claim 9, and represented by formula (lVa) 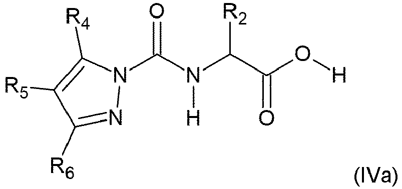 wherein
R₂ is selected from -H, -OH, -SH, SeH, -NR_{b}R_{b'}, -C₁₋₁₂alkyl, -C₂₋₁₂alkenyl, -C₃₋₇cycloalkyl, - CHO, -C(O)R_{c}, -COOH, -OC(O)R_{c}, -C(O)OR_{c}, -OC(O)OR_{c}, -NR_{b}C(O)R_{c}, -C(O)NR_{b}R_{b'}, - NR_{b}C(O)OR_{c}, and -OC(O)NR_{b}R_{b'}-, and Ar₂; wherein said -C₁₋₁₂alkyl, -C₂₋₁₂alkenyl, and -C₃₋₇cycloalkyl optionally comprises one or more heteroatoms selected from N, O, and S and/or is substituted with one or more substituents selected from -H, -OH, -SH, -NR_{d}R_{d'}, -C₁₋₁₂alkyl, -C₂₋₁₂alkenyl, -C₃₋₇cycloalkyl, -CHO, -C(O)Rₑ, -COOH, -OC(O)Rₑ, -C(O)ORₑ, -OC(O)ORₑ, - NR_{d}C(O)Rₑ, -C(O)NR_{d}R_{d'}, -NR_{d}C(O)ORₑ, -OC(O)NR_{d}R_{d'}-, and Ar₃;
R₄ and R₆ are each independently selected from -H, -halogen, -C₁₋₁₂alkyl, -C₂₋₁₂alkenyl, -C₃₋₇cycloalkyl, and Ar₄; wherein said -C₁₋₁₂alkyl, -C₂₋₁₂alkenyl, and -C₃₋₇cycloalkyl optionally comprises one or more heteroatoms selected from N, O, and S and/or is substituted with one or more substituents selected from -halogen;
R₅ is selected from -H, -OH, -SH, -NR_{g}R_{g'}, -C₁₋₁₂alkyl, -C₂₋₁₂alkenyl, -C₃₋₇cycloalkyl, -CHO, - C(O)Rₕ, -COOH, -OC(O)Rₕ, -C(O)ORₕ, -OC(O)ORₕ, -NR_{g}C(O)Rₕ, -C(O)NR_{g}R_{g'}, - NR_{g}C(O)ORₕ, and -OC(O)NR_{g}R_{g'}-, and Ar₅; wherein said -C₁₋₁₂alkyl, -C₂₋₁₂alkenyl, and -C₃₋₇cycloalkyl optionally comprises one or more heteroatoms selected from N, O, and S and/or is substituted with one or more substituents selected from -H, -OH, -SH, -NRᵢR_{i'}, -C₁₋₁₂alkyl, -C₂₋₁₂alkenyl, -C₃₋₇cycloalkyl, -CHO, -C(O)Rⱼ, -COOH, -OC(O)Rⱼ, -C(O)ORⱼ, -OC(O)ORⱼ, - NRᵢC(O)Rⱼ, -C(O)NRᵢR_{i'}, -NRᵢC(O)ORⱼ, -OC(O)NRᵢR_{i'}-, and Ar₆;
Ar₂, Ar₃ Ar₄, Ar₅, and Ar₆ are each independently a 5- to 10-membered aromatic cycle optionally comprising one or more heteroatoms selected from O, N and S and/or substituted with one or more substituents independently selected from -halogen, -C₁₋₆alkyl, -C₂₋₆alkenyl, -C₃₋₇cycloalkyl, -OH, -SH, NR_{f}R_{f'}, and -OC₁₋₆alkyl;
each instance of R_{b}, each instance of R_{b'}, each instance of R_{c}, each instance of R_{d}, each instance of R_{d'}, each instance of Rₑ, each instance of R_{g}, each instance of R_{g'}, each instance of Rₕ, each instance of Rᵢ, each instance of R_{i'} and each instance of Rⱼ is independently selected from -H, and -C₁₋₁₂alkyl; and
each instance of R_{f} and each instance of R_{f'} is independently selected from -H, and -C₁₋₆alkyl.

11. A method for preparing a protected dipeptide, the method comprising the steps of:
a) providing a protected amine as defined in claim 9 or 10; and
b) coupling the protected amine provided in step a) with a further alpha-amino acid compound, thereby obtaining the protected dipeptide.

12. Method for preparing a protected oligopeptide or protected polypeptide, the method comprising the steps of preparing the protected dipeptide as claimed in claim 11, and comprising the further steps of:
c) deprotecting the N-terminus of the protected dipeptide, obtained in step b), by contacting the protected dipeptide with a source of fluoride or hydroxide soluble in organic media, and subsequently with a nucleophile;
d) coupling the deprotected dipeptide obtained in step c) with a further alpha-amino acid compound, thereby obtaining the protected oligopeptide; and
e) optionally repeating steps c) and d) one or more times to add further amino acid residues to the protected oligopeptide obtained in step d).

13. Method for preparing an oligopeptide or polypeptide with an unprotected N-terminus, the method comprising the steps of preparing the protected oligopeptide or protected polypeptide as claimed in claim 12, and comprising the further steps of:
f) contacting the protected oligopeptide or protected polypeptide obtained in step d) or step e) with a source of fluoride or hydroxide soluble in organic media; and
g) contacting the compound obtained in step f) with a nucleophile.

14. Method for preparing an oligopeptide or polypeptide with an unprotected N-terminus as claimed in claim 13, wherein the nucleophile is present when the protected oligopeptide or protected polypeptide is contacted with the source of fluoride or hydroxide soluble in organic media and/or the nucleophile is added after the protected oligopeptide or protected polypeptide is contacted with the source of fluoride or hydroxide soluble in organic media.

15. A protected oligopeptide represented by formula (V) 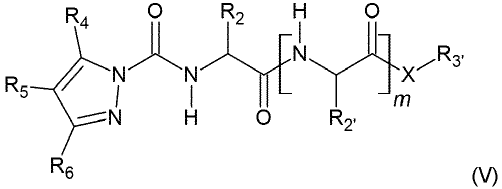 wherein
m is an integer from 1 to 100;
R₂ and each instance of R_{2'} are each independently selected from -H, -OH, -SH, SeH, - NR_{b}R_{b'}, -C₁₋₁₂alkyl, -C₂₋₁₂alkenyl, -C₃₋₇cycloalkyl, -CHO, -C(O)R_{c}, -COOH, -OC(O)R_{c}, - C(O)OR_{c}, -OC(O)OR_{c}, -NR_{b}C(O)R_{c}, -C(O)NR_{b}R_{b'}, -NR_{b}C(O)OR_{c}, and -OC(O)NR_{b}R_{b'}-, and Ar₂; wherein said -C₁₋₁₂alkyl, -C₂₋₁₂alkenyl, and -C₃₋₇cycloalkyl optionally comprises one or more heteroatoms selected from N, O, and S and/or is substituted with one or more substituents selected from -H, -OH, -SH, -NR_{d}R_{d'}, -C₁₋₁₂alkyl, -C₂₋₁₂alkenyl, -C₃₋₇cycloalkyl, - CHO, -C(O)Rₑ, -COOH, -OC(O)Rₑ, -C(O)ORₑ, -OC(O)ORₑ, -NR_{d}C(O)Rₑ, -C(O)NR_{d}R_{d'}, - NR_{d}C(O)ORₑ, -OC(O)NR_{d}R_{d'}-, and Ar₃;
each instance of R_{b}, each instance of R_{b'}, each instance of R_{c}, each instance of R_{d}, each instance of R_{d'} and each instance of Rₑ is independently selected from -H, and -C₁₋₁₂alkyl; Ar₂ and Ar₃ are each independently a 5- to 10-membered aromatic cycle optionally comprising one or more heteroatoms selected from O, N and S and/or substituted with one or more substituents independently selected from -halogen, -C₁₋₆alkyl, -C₂₋₆alkenyl, -C₃₋₇cycloalkyl, -OH, -SH, NR_{f}R_{f'}, and -OC₁₋₆alkyl;
each instance of R_{f} and each instance of R_{f'} is independently selected from -H, and -C₁₋₆alkyl;
X is selected from -O-, and -NH-;
R_{3'} is selected from -H, and -C₁₋₁₂alkyl, or R_{3'} is a solid support;
R₄, and R₆ are each independently selected from hydrogen, any halogen, any substituted or unsubstituted alkyl, any substituted or unsubstituted alkenyl, any substituted or unsubstituted cycloalkyl, and any substituted or unsubstituted aryl; and
R₅ is selected from hydrogen, hydroxy, thio, any substituted or unsubstituted amino, any substituted or unsubstituted alkoxy or aryloxy, any substituted or unsubstituted alkyl, any substituted or unsubstituted alkenyl, any substituted or unsubstituted cycloalkyl, any substituted or unsubstituted aryl, any substituted or unsubstituted ester, any substituted or unsubstituted carbonyl, any substituted or unsubstituted amide, any substituted or unsubstituted carbonate, and any substituted or unsubstituted carbamate.
